# EUROPEAN PATENT SPECIFICATION

(11) **EP 1 219 628 B1**
(45) Date of publication and mention of the grant of the patent: **02.04.2003**
(21) Application number: 00128552.7
(22) Date of filing: 27.12.2000
(51) Int. Cl.: C07F 9/572, C12Q 1/34, C12Q 1/04

(54) **Novel substrates for detection of microbial metabolites**
Neue Substrate zur Detektion von mikrobiellen Metaboliten
Nouveaux substrats pour la détection de métabolites microbiens

(43) Date of publication of application: 03.07.2002
(73) Proprietor: Biosynth AG, 9422 Staad (CH)
(72) Inventor: Schabert, Günter, Dr., 9403 Goldach (CH); Restaino, Lawrence, Dr., West Chigago, IL 60185 (US); Frampton, Elon, W., Dr., Dekalb, IL 60115 (US)
(74) Representative: Ritscher, Thomas, Dr.

(56) References cited:
- WO-A-98/38332
- WO-A-99/50438
- US-A- 4 082 781
- KURIOKA S. ET AL.: "Phospholipase C assay using p-nitrophenylphosphorylcholine together with sorbitol and its application to studying the metal and detergent requirement of the enzyme" ANALYTICAL BIOCHEMISTRY., vol. 75, 1976, pages 281-289, XP001002863 ACADEMIC PRESS INC. NEW YORK., US ISSN: 0003-2697

## Description

### Field of the Invention

The present invention relates to novel compounds and substrates which become colorimetrically detectable upon contact with certain microorganisms or microbial metabolites, i.e. substances secreted or otherwise produced by such microorganisms, as well as to the use of such compounds and substrates for detection and identification of various microorganisms including bacteria.

### Prior Art

Phospholipase C enzymes are found in a variety of microbes. These enzymes have been associated with the pathogenicity of the microbes to its host.

More specifically, it is known that the enzyme termed "Phosphatidylcholine specific Phospholipase C (also known as Phosphatidylcholine cholinephosphohydrolase or Lecithinase C, also termed PC-PLC herein for short; enzyme classification EC 3.1.4.3) can be found in a variety of microbes including *Clostridium perfringens, Clostridium novyi, Bacillus cereus, Bacillus thuringiensis, Pseudomonas aeruginosa* and *Staphylococcus aureus* (cf. J.G. Songer; Trends in Microbiology 5 (1997), 156) as well as *Helicobacter pylori* (cf. J.-H. Weitkamp et al.; Zentralblatt für Bakteriologie 280 (1993), 11), *Legionella pneumophila* (cf. W.B. Baine; Journal of General Microbiology 134 (1988), 489), and *Listeria monocytogenes* (cf. A. Coffey et al.; Applied and Environmental Microbiology 62 (1996), 1252). Furthermore, PC-PLC has been found in yeasts, e.g. *Candida albicans* (cf. M. Birch et al.; Infect. Immun. 64 (1996), 751).

Several procedures for assay of PC-PLC have been developed. Some of the more recent assays were reviewed by E.L. Krug and C. Kent (cf. Methods in Enzymology 72 (1981), 347). The most commonly used procedures detect choline phosphate produced by the phospholipase C reaction on the natural substrate phosphatidylcholine. For other methods special equipment is needed. All these methods allow only measurement of the total amount of enzyme present in a sample at a certain moment and therefore are discontinuous assay methods. Additionally, all these procedures are clearly not suitable for the direct detection of microbes secreting PC-PLCs.

In 1976, Kurioka et al. (cf. S. Kurioka, M. Matsuda; Analytical Biochemistry 75 (1976), 281) have reported a continuous spectrophotometric assay for PC-PLC using a substrate containing 4-Nitrophenyl choline phosphate (p-Nitrophenylphosphorylcholine). Kurioka first synthesized this compound in 1968 (cf. S. Kurioka; Journal of Biochemistry 63 (1968), 678).

However, this substrate has several disadvantages. The specific activity of the enzyme towards this substrate is extremely low. Only after the addition of sorbitol or glycerol in high concentrations (up to 60 %) an assay of PC-PLC with reasonable, yet still low cleavage rates could be developed. Thus, as already stated by Krug and Kent, this procedure is only suitable for investigations with pure enzyme preparations. This means that this substrate is less suitable for direct detection of microbes or microbial secretes containing PC-PLCs.

Furthermore this substrate cannot be used for plating media since the 4-nitrophenolate liberated upon enzymic cleavage is water-soluble and thus would migrate into the medium. Additionally the yellow color of 4-Nitrophenolate may interfere with the background in biological samples like body fluids or culture media.

Generally, prior art assay methods are unspecific, not flexible and do not allow continuous measurement of actual PC-PLC concentrations in a sample.

Prior art methods for detection and identification of bacteria producing PC-PLC use freshly prepared egg yolk agar. Egg yolk contains a variety of phosphatides; the main constituents are phosphatidylcholine, phosphatidylethanolamine and inositol phosphatides. Phosphatidylcholine is cleaved by PC-PLC to form choline phosphate and water-insoluble diglycerides, the latter giving an opaque zone around the bacterial colonies producing PC-PLC on egg yolk agar.

These methods were improved by the use of Lecithin agar (cf. G.L. Chrisope et al.; Applied and Environmental Microbiology 31 (1976), 784) containing crude soybean lecithin. Bacteria secreting PC-PLC showed turbid halos around the colonies indicating the presence of the enzyme. The average time to produce a reaction of moderate degree was about two to three days. This detection method lacks specificity because other phospholipases, e.g. phospholipase A or phosphatidylinositol-specific phospholipase C (PI-PLC) may act on other components of lecithin producing opalescent zones around the colony, too. Furthermore, training is required to correctly screen the plates for such zones. Generally, these prior art methods are unspecific, laborious and time consuming and, hence, expensive.

US-A-4 082 781 discloses a relatively complicated method of preparing 2-alkanoylamino-4-nitrophenyl-phosphorylcholine hydroxides suitable in diagnostic testing for Niemann-Pick disease.
WO-A-98 383 32 discloses substrates for assay of phosphatidylinositol specific phospholipase C (PI-PLC), as well as a method for the detection of that enzyme. These substrates can so be used for detection of potentially pathogenic bacteria producing PI-PLC enzymes.
WO-A-99 504 38 describes a large number of indoxyl substrates, many of which were disclosed previously but fails to anticipate or render obvious the present invention.

In sum, prior art does not disclose a simple plating medium capable of discriminating between *Bacillus cereus* and *Bacillus thuringiensis*, in particular since data show that *Bacillus cereus* and *Bacillus* *thuringiensis* should be regarded as one species (cf. C.R. Carlson, Applied and Environmental Microbiology 60 (1994), 1719) where horizontal transfer of plasmid genome occurs.

### Objects and Summary of the Invention

Accordingly it is a primary object of the present invention to provide novel chromogenic compounds for easy and convenient detection of PC-PLC, which avoid the disadvantages of prior art.

It is another main object of the present invention to provide novel chromogenic substrates and methods for detection and/or identification of microorganisms producing PC-PLC by means of conventional spectrophotometric and/or histochemical assays including use in broth and especially in plating media and which substrates are substantially free from the disadvantages of prior art substrates or methods.

It is a further object of the present invention to provide means for detecting and/or identifying various pathological bacteria, such as *Clostridium perfringens, Pseudomonas aeruginosa, Helicobacter pylori, Legionella pneumophila, Bacillus cereus* and others.

Yet another object of the present invention is a method to discriminate *Bacillus cereus* from *Bacillus thuringiensis.*

The above and further objects and advantages as apparent from the specification will be achieved when use is made of novel chromogenic compounds of formula I: 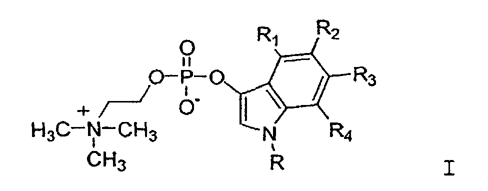 in which R is selected from the group consisting of hydrogen and C₁₋₄ alkyl, such as methyl, ethyl, and all possible isomers of propyl and butyl, while R¹, R², R³, and R⁴ are radicals independently selected from the group consisting of hydrogen, halogen (e.g. fluorine; chlorine, bromine and iodine), cyano, nitro, carboxy, amino, amino . substituted with one or two C₁₋₄ alkylgroups, aminomethyl, hydroxy, C₁₋₄ alkoxy, carboxyalkyl, and sulphonyl.

The term "chromogenic" as used herein with reference to the novel compounds according to the invention indicates the capability of these compounds to become colored, i.e. visible or colorimetrically detectable, upon interaction with bacteria and in particular upon interaction with PC-PLC.

The terms "detection" or "detecting" are intended herein to include detection methods and assay techniques, as well as substances or substrates for use in such methods or techniques.

As will be apparent to those experienced in the art, the most preferred compounds within the scope of formula I above are those which yield deeply colored indigo dyes when used as substrates for detecting PC-PLCs, and which are, preferably, easily accessible and well suited for histochemical uses. Examples of preferred formula I compounds in accordance with the present invention are given below.

While no theoretical limitation is intended, the effectiveness of compounds of formula I as substrates for detection of PC-PLC enzymes resides in the fact that cleavage of a compound according to the invention by bacterial PC-PLCs results mainly in the formation of choline phosphate and (optionally substituted) Indoxyl (3-Hydroxy-indole). The (optionally substituted) Indoxyl then quickly dimerizes and is subsequently oxidized by atmospheric oxygen or another oxidant to a deeply colored indigo dye, which is a well-known chromophor, suitable for sensitive colorimetric detection by conventional methods and apparatus.

On plating media Indigo dyes give characteristic, strongly colored precipitates, which are water-insoluble. Therefore the color stays with the colony and does not diffuse throughout the plate. Also, the color is clearly distinguishable from even a yellow background of the medium. Thus, the novel compounds and substrates for PC-PLCs are very well suited for histochemical uses. As is shown below, media using the novel substrates and compounds of formula (I) will generally improve and facilitate detection and even identification of PC-PLC producing colonies of bacteria and other microorganisms.

According to a second aspect, the present invention provides for a substrate for detecting a phosphatidylcholine-specific Phospholipase C enzyme as an indication of microbial or bacterial activity. The substrate comprises at least one inventive chromogenic 3-Indoxyl choline phosphate compound of formula I, preferably being in a suitable medium, such as aqueous agar-agar.

The term "substrate" as used herein and, particularly as compared to the inventive compounds, indicates that for the purpose of detecting PC-PLC the at least one inventive formula I compound is typically used and applied while being in a suitable media, such as aqueous agar-agar or conventional buffer solutions.

According to a third aspect, the invention provides for a method of detecting a Phosphatidylcholine-specific Phospholipase C enzyme comprising the steps of contacting a sample suspected of containing the phosphatidylcholine-specific Phospholipase C enzyme with an inventive substrate containing at least one compound of formula I, wherein, as indicated above, the at least one compound is susceptible to cleavage by the PC-PLC enzyme yielding a dye; and monitoring for color formation as a consequence of the PC-PLC enzyme suspected of being contained in the sample.

According to a fourth aspect, the invention provides for a method of detecting microbial activity in a sample by combining the sample with an inventive substrate or a compound of formula I and inspecting the resulting mixture by spectroscopic means, in particular colorimetric means.

According to a fifth aspect, the invention provides for a kit for detecting PC-PLC as an indication of bacterial or microbial activity; the kit includes at least one compound of formula I.

According to further aspects, the invention provides for a continuous assay of PC-PLC by spectrophotometric means as well as for assay methods for detection of the presence of pathogenic strains of *Clostridium perfringens*, or of *Bacillus cereus* or *Bacillus thuringiensis*, or of *Pseudomonas aeruginosa* in a sample suspected to contain such strains by means of an inventive substrate.

According to a yet further aspect, the invention provides for a method of identifying a bacterial microorganism of interest, which is capable of producing a phosphatidylcholine-specific phospholipase C (PC-PLC) enzyme comprising the steps of: (A) providing a test sample suspected of containing the microorganism of interest; (B) preferably submitting the test sample to an enrichment broth step; (C) transferring a portion, at least, of the test sample or the product obtained in step (B) to a medium suitable for culturing the microorganism; the medium containing at least one compound of formula I capable of producing a color when exposed to the microorganism; (D) cultivating the medium with the transferred portion for developing at least one colony exhibiting said color; and (E) recovering a portion, at least, of the colored colony for final identification.

According to another aspect, the invention provides for a method of producing a compound of formula I. In the first step of the process, the corresponding (optionally N-protected) Indoxyl-3-dichlorophosphate, i.e. the compound of formula II, is reacted with a salt of choline, such as choline chloride or choline iodide, to obtain the intermediate compound of formula III given below, e.g. by stirring the reactants in an organic base, such as quinoline, triethylamine, N-methylmorpholine or pyridine as a reaction medium at ambient temperature during a period of several hours (e.g. 1-8 hours): 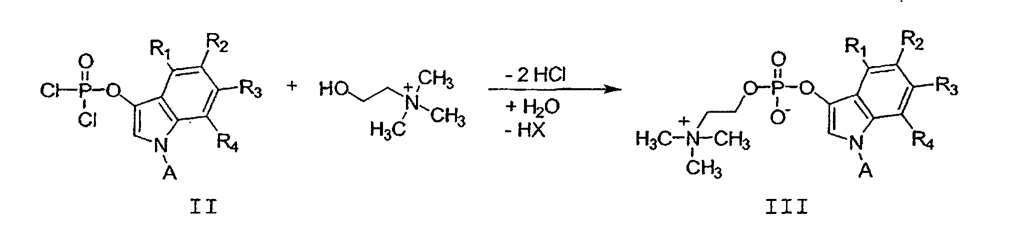 in which A represents hydrogen or a conventional protecting group for nitrogen, such as C₁₋₄ alkyl (preferably methyl), acyl (preferably acetyl or benzoyl) or N-protecting groups known from peptide chemistry as Boc, CBZ , Fmoc etc.; and wherein X is an anion derived from an inorganic or organic acid, e.g. hydrochloric acid; and R¹ - R⁴ have the meaning indicated above for formula I.

To complete the synthesis of the compounds of formula I, in the subsequent second process step, the optional N-protecting group on the intermediate of formula III is removed - e.g. by acidic or mild basic cleavage or hydrogenolysis, depending upon the nature of the group or by other conventional methods known from peptide chemistry - and optionally the group R is introduced by conventional reaction.

From the above it will be apparent that compounds of formula I including the preferred ones enumerated below can be manufactured efficiently in sufficiently large quantities as are required for use as substrates for application in standard screening procedures or plating media.

According to a yet further aspect, the invention provides for a method of preparing a substrate capable of detecting a microbial Phosphatidylcholine-specific Phospholipase C enzyme wherein the method comprises the step of producing the substrate by incorporating therein a 3-Indoxyl choline phosphate compound of formula I.

### Preferred Embodiments of the Invention

For high chromogenicity, in a preferred group of formula I compounds, R is selected from hydrogen or methyl, R¹ is selected from the group consisting of hydrogen and halogen (chlorine and fluorine being preferred), R² is selected from the group consisting of hydrogen, cyano, nitro and halogen (bromine and iodine being preferred), and R³ and R⁴ are independently selected from the group consisting of hydrogen and halogen (chlorine and fluorine being preferred).

A very preferred specific novel compound is 5-Bromo-4-chloro-3-indoxyl choline phosphate, i.e. the compound of formula IV below:

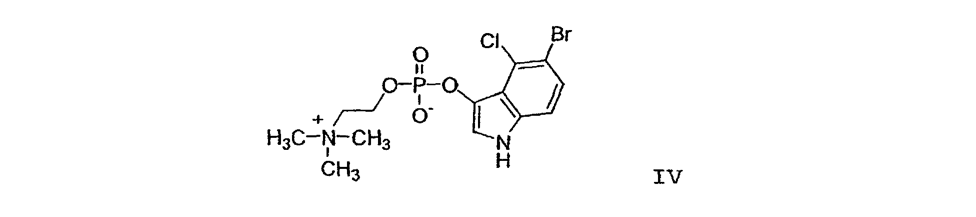

The compound of formula IV will be referred to as "X-phos-choline" or "X-CP" herein below.

Another very specific novel compound is 3-Indoxyl choline phosphate, i.e. the compound of formula V below:

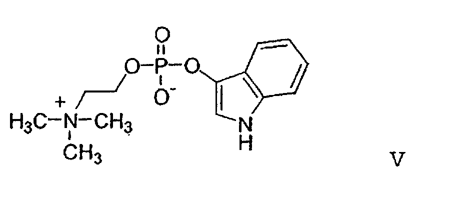

The compound of formula V will be referred to as "Y-phos-choline" or "Y-CP" herein below.

Both compounds are white, crystalline powders being freely soluble in water. Tests made with X-phos-choline (X-CP) and Y-phos-choline (Y-CP) showed that the novel compounds and substrates are stable during extended periods at temperatures below about 5°C while protected from light. Thus, the same properties can be safely expected from the other compounds of formula I.

Furthermore, the novel compounds and substrates, based upon the tests made with X-phos-choline and Y-phos-choline, proved to be very stable in conventional buffer solutions (Citrate/Hydrochloric acid; Hepes/NaOH; Tris/HCl; Boric acid/potassium chloride-sodium hydroxide) for at least ten days at a pH ranging from 4 to 10 and ambient temperature as well as in conventional plating media, e.g. Tryptic Soy Agar. Thus, problems with background signals caused by slow non-enzymic hydrolysis in the buffer media as observed with the prior art substrate 4-Nitrophenyl choline phosphate are avoided. Again, similar properties can be expected for the other formula I compounds.

The 5,5'-Dibromo-4,4'-dichloroindigo generated from cleavage of X-CP by PC-PLCs, dimerization and subsequent oxidation is a dye known per se and has a broad absorption ranging from ≈ 500 nm to ≈ 700 nm with its maximum at 652 mm. The indigo dye has an intense brilliant blue color with an absorption coefficient near 6000 L mol⁻¹ cm⁻¹.

A further very preferred group of compounds of formula I which are particularly suitable for the purposes of the present invention are: 4-Chloro-3-indoxyl choline phosphate, 5-Bromo-3-indoxyl choline phosphate, 5-Bromo-6-chloro-3-indoxyl choline phosphate (referred to as "Magenta-CP" herein below), 6-Chloro-3-indoxyl choline phosphate (referred to as "Salmon-CP" herein below) and 6-Fluoro-3-indoxyl choline phosphate. In particular, Magenta-CP and Salmon-CP are specific within this group since their cleavage by PC-PLCs, dimerization and subsequent oxidation lead to dies of characteristic red colors.

In accordance with an important aspect of the present invention the novel compounds and substrates are used for a continuous spectrophotometric assay of PC-PLC, e.g. from *Clostridium perfringens*. As indicated above, the addition of sorbitol or glycerol in high concentrations (up to 60 %) to the prior art 4-Nitrophenyl choline phosphate accelerates the enzymatic cleavage of 4-Nitrophenyl choline phosphate by *Clostridium perfringens* PC-PLC, and thus leading to an assay for detection of PC-PLC with reasonable, but still low cleavage rates.

On the other hand,- the addition of deoxycholic acid, glycerol or especially sorbitol to the substrates of the present invention did not improve the enzymatic reaction on the novel compounds of formula I but even act in an inhibitory way. Moreover, raising the temperature to about 60°C did not accelerate the enzymatic hydrolysis markedly though the temperature optimum for the cleavage of the prior art substrate 4-Nitrophenyl choline phosphate by PC-PLC of *Clostridium perfringens* is reported at 65°C (cf. J.-H. Weitkamp; Zentralblatt für Bakteriologie 280 (1993), 15).

Unexpectedly it has been found that the cleavage rate is considerably enhanced when use is made of at least one additive selected from the group consisting of serum albumin, surfactants and metal ions, wherein the metal ion is preferably selected from a divalent ion of cobalt, manganese, nickel, zinc, calcium or magnesium.

In a further preferred embodiment of the present invention the substrate further comprises at least one metal ion and at least one member selected from the group consisting of surfactants and serum albumin, wherein, preferably, the metal ion is a divalent ion of cobalt, manganese, nickel, zinc, calcium or magnesium.

The preferred selected additive or the combination of additives depend on the microorganism to be detected. It is important to note that the preferred additive or combination enhanced the enzymatic cleavage not only by a factor of 2 or 3 as did additives with the prior art substrates (cf. (cf. J.-H. Weitkamp et al.; Zentralblatt für Bakteriologie 280 (1993), 11 and W.B. Baine; Journal of General Microbiology 134 (1988), 489) but by a factor of at least 100.

This is surprising since at least for the "natural substrates" of PC-PLC, e.g. the phosphatidylcholines, a certain amount of phospholipid chain hydrophobic binding seemed to be important for this water-soluble surface-active enzyme. Moreover, fatty acid carbonyls (preferably unhindered) are described to be more critical to productive binding and subsequent hydrolysis (cf. El-Sayed et al; Biochimica et Biophysica Acta 837 (1985), p. 326). Furthermore, El-Sayed et al. have indicated that fatty acid chains must be sufficiently long to produce a hydrophobic binding site on the lipid molecule (cf. El-Sayed et al.; Biochimica et Biophysica Acta 837 (1985), p. 333). However, the novel compounds in accordance with the invention are very polar and lack any hydrophobic sites, e.g. longer alkyl chains, as well as fatty acid carbonyls.

Thus, using a preferred and proper additive or combination of additives, the inventive novel substrates unexpectedly can be utilized for sensitive assay of PC-PLC from a particular microorganism. Typically, the preferred suitable additive or combination of additives is evaluated individually for each microorganism to be detected or each PC-PLC to be assayed. This can be done by simple experiments, e.g. as shown in the example below.

Typically, the inventive compound is used in combination with at least one enhancer selected from bovine serum albumin (BSA), certain divalent metal ions, e.g. of cobalt, manganese, nickel, zinc, magnesium or calcium, and a surfactant, preferably a nonionic surfactant, such as polysorbates and very preferably Tween 80 (polyoxyethylene sorbitan monooleate), depending on the PC-PLC of the microorganism to be assayed.

While no theoretical limitation is intended, the special effect of BSA is believed to result from the hydrophobic nature of the protein, which is known to enhance reactions of enzymes that normally act on hydrophobic substrates, e.g. PC-PLC enzymes cleaving phosphatidylcholine.

In a very preferred embodiment of the present invention the substrate comprises at least one compound of formula I, preferably X-CP, and a combination of BSA and a divalent salt of cobalt, preferably cobalt(II)sulfate. This very preferred combination has been found to extremely enhance the cleavage of X-CP by *Clostridium perfringens* PC-PLC. Thus, using this combination, together with X-CP, a sensitive determination of PC-PLC from *Clostridium perfringens* is now possible.

Compared, in particular, to the prior art substrate 4-Nitrophenyl choline phosphate, the inventive novel compounds and substrates are clearly advantageous since assay of PC-PLC is possible in ordinary buffer solutions, preferably by adding just small amounts of additives, whereas with 4-Nitrophenyl choline phosphate reasonable velocity of enzymatic hydrolysis required the use of viscous solutions of this prior art substrate in sorbitol.

A further important aspect of the present invention deals with the use of compounds of formula I, e.g. the very preferred 5-Bromo-4-chloro-3-indoxyl choline phosphate (X-phos-choline; X-CP) or 3-Indoxyl choline phosphate (Y-phos-choline; Y-CP) for the detection of PC-PLC in microorganisms, preferably by spectroscopic means, and very preferably by colorimetric means.

The data in the examples presented below clearly show that the chromogenic substrates X-CP and Y-CP can be used to detect PC-PLC by the formation of a turquoise to blue colony color on conventional plating media. The color stays within the colony as the insolubility prevents migration of the dye to the medium.

Further preferred embodiments of the present invention are described in the dependent claims.

As outlined above, PC-PLCs are produced by a variety of human pathogens. Therefore the invention provides a method for detecting such pathogens by means of the novel compounds and substrates, e.g. by screening for bacterial enzyme production directly on plating media, for example, of clinical samples or cultures isolated from food.

### Brief Description of the drawings

The invention will now be explained in more detail by way of examples and with reference to the enclosed drawings in which:
- Fig. 1: is a graph showing dependence of the absorbance (on the ordinate) upon time (on the abscissa) at various temperatures obtained with the inventive substrate comprising X-phos-choline (X-CP) upon cleavage with PC-PLC from *Clostridium perfringens* (cf. Example 4);
- Fig. 2: is a graph similar to that of Fig. 1 except that the curves are shown at various concentrations of X-CP at a temperature of 58-59°C (cf. Example 5);
- Fig. 3: is a Lineweaver-Burk plot of 1/Cleavage Rate (ordinate; in min/nMol) versus 1/X-CP concentration (abscissa; in 1/mM) (cf. Example 5);
- Fig. 4: is a graph showing dependence of the absorbance (ordinate) upon time (abscissa) at 0.01, 0.02, 0.05, 0.1 and 0.2 Units of *Clostridium perfringens* PC-PLC obtained with a substrate comprising X-CP at a temperature of 58-59°C (cf. Example 6);
- Fig. 5: is a graph similar to that of Fig. 4, however for 0.5, 1, 2, 4 and 8 Units of enzyme;
- Fig. 6: is a graph showing the rate of cleavage of X-CP (ordinate; in nMol/min) versus amount of enzyme (abscissa; in µg) in the range of 0.01 to 0.5 Units (i.e. 33 ng to 1667 ng) of *Clostridium perfringens* PC-PLC (cf. Example 6); and
- Fig. 7: shows a linear relationship between the rates of cleavage of X-CP(ordinate) and the expression ^{1,6}√ amount of Enzyme (abscissa) in the range of 0.5 to 8 Units (i.e. 1.66 to 27 µg) of enzyme (Example 6).

It is to be noted, however, that the specific examples are not intended to limit the invention in any way.

### EXAMPLES

### Preparation of the new compounds of formula I

1-Acetyl-5-bromo-4-chloro-3-indoxyl-dichlorophosphate (1-Acetyl-5-bromo-4-chloro-3-indoxyl-phosphorodichloridate) was prepared as described in the literature (cf. J.P. Horwitz, J.V. Freisler; Journal of Medicinal Chemistry 13 (1970), 1024). 1-Acetyl-3-indoxyl-dichlorophosphate was prepared analogously. Choline iodide was synthesized according to the procedure of B. Chesebro and H. Metzger (cf. Biochemistry 11 (1972), 766).

### Example 1: Preparation of X-phos-choline (IV)

### Step 1: Preparation of 1-Acetyl-5-bromo-4-chloro-3-indoxyl choline phosphate

1-Acetyl-5-bromo-4-chloro-3-indoxyl-dichlorophosphate (4.06 g, 10 mMol) was suspended under nitrogen in dry acetonitrile (12 ml) and choline iodide (2.31 g; 10 mMol) was added. The mixture was stirred at ambient temperature while quinoline (1.46 ml; 10 mMol) was added dropwise over five minutes. The choline iodide slowly dissolved and after an hour a slightly turbid solution was obtained, which was stirred at the same temperature for a period of another 2 hours.

After the solution had been filtered from a little solid matter, the clear, brownish-yellow filtrate was added dropwise to a cooled (0-5°C) solution of pyridine (5.6 ml; 70 mMol) in water (20 ml).

The solution was evaporated under reduced pressure to a yellow-brown oil, which was dissolved in water (60 ml) and mixed-bed exchange resin MB-150 (Sigma # A-5710; 60 g) was added and stirred for a period of 20 minutes. The pH was adjusted to ≈ 5 by addition of a 25 % ammonium hydroxide solution in water (1.5 ml) and the resin was removed by filtration through a glass filter funnel and washed with water.

To prevent foaming, ethanol (10 ml) was added to the filtrate and the solution was cautiously concentrated by rotary evaporation under reduced pressure to a volume of 40 ml. Upon cooling to ambient temperature colorless crystals of the product appeared. The suspension was stored overnight at 5°C. The product was collected by filtration and washed twice with ice-water (2 x 2 ml) and once with acetone (5 ml) and finally dried in vacuum to give 1.28 g of a white, crystalline powder. From the mother liquor and the acetone washing another 0.20 g and 0.24 g of material was obtained, respectively. Overall yield 1.72 g (37 %).
m.p. 241-243°C (dec.).
Analysis calcd. for C₁₅H₁₉BrClN₂O₅P (MW= 453.65): C 39.71, H 4.22, N 6.18, Br 17.61, Cl 7.82, P 6.83; Found (calcd. on dry matter; water content 3.95 %): C 39.70, H 4.25, N 6.12, Br 17.49, Cl 7.83, P 6.72;
¹H-NMR (400 MHz; DMSO-d₆), δ (ppm): 8.22 (d; 1H), 7.65 (broadened s, 1H), 7.62 (d; 1H), 4.18 (m, 2H), 3.55 (m, 2H), 3.11 (s, 9H), 2.56 (s, 3H).
¹³C-NMR (400 MHz; DMSO-d₆), δ (ppm): 169.0, 135.8 (d, J_{P,C}= 6.4 Hz), 132.6, 129.3, 123.8, 123.2 (d, J_{P,C}= 6.6 Hz), 116.9, 116.0, 113.6 (small d, J_{P,C}= 2.4 4 Hz), 65.4 (m), 59.0 (d, J_{P,C}= 5.6 Hz), 53.1 (t, J_{N,C}= 3.5 Hz), and 23.7.

### Step 2: Preparation of 5-Bromo-4-chloro-3-indoxyl choline phosphate (IV)

1-Acetyl-5-bromo-4-chloro-3-indoxyl choline phosphate (0.51 g; 1.1 mMol) was dissolved in a 2 N solution of ammonia in methanol (10 ml) under nitrogen. The educt quickly dissolved leaving a greenish-yellow solution. After a period of 2 hours the solvent was removed in vacuo.

The greenish-beige foam thus obtained was dissolved in. warm (≈ 50°C) ethanol (2 ml) and acetone (4 ml) was added. The solution was seeded whereby a few minutes later a white, crystalline precipitate appeared. The suspension was stirred for half an hour at ambient temperature, whereupon additional acetone (1 ml) was added. An hour later the suspension was filtered through a glass filter funnel and the crystals washed with acetone (2 x 2 ml). The product was dried in vacuo leaving 0.36 g (80 %) of a nearly colorless, crystalline powder.
m.p. 247-248°C (dec.).
Analysis calcd. for C₁₃H₁₇BrClN₂O₄P (MW= 411.62) : C 37.93, H 4.16, N 6.80, Br 19.41, Cl 8.61, P 7.52; Found (calcd. on dry matter; water content 5.3 %) C 37.90, H 4.20, N 6.82, Br 19.23, Cl 8.48, P 7.40;
¹H-NMR (400 MHz; D₂O), δ (ppm): 7.22 (small d; 1H), 7.19 (d, 1H), 7.06 (d; 1H), 4.28 (broad s, 2H), 3.43 (t, 2H, J_{P,H}= 4.4 Hz), 2.95 (s, 9H) ;
¹³C-NMR (400 MHz; D₂O), δ (ppm): 133.5, 129.9 (d, J_{P,C}= 7.5 Hz), 126.9, 123.1, 118.4 (d, J_{P,C}= 4.9 Hz), 115.7 (broadened s), 113.4, 112.8, 66.5 (m), 60.5 (d, J_{P,C}= 5.2 Hz), 54.2 (broadened s).
UV (0.1 N HEPES/NaOH pH 7.0) : λₘₐₓ = 290 nm, ε = 4910 L Mol⁻¹ cm⁻¹.

### Example 2: Preparation of 3-Indoxyl choline phosphate (V)

### Step 1: Preparation of 1-Acetyl-3-indoxyl choline phosphate

1-Acetyl-3-indoxyl-dichlorophosphate (8.76 g, 30 mMol) was suspended under nitrogen in dry acetonitrile (36 ml) and choline iodide (6.93 g; 30 mMol) was added. The mixture was stirred at ambient temperature while quinoline (4.38 ml; 30 mMol) was added dropwise over fifteen minutes. The choline iodide slowly dissolved and after half an hour a slightly turbid solution was obtained, which was stirred at the same temperature for another hour. The brownish-yellow solution was then added dropwise to a cooled (0-5°C) solution of pyridine (16.8 ml; 210 mMol) in water (60 ml).

The solution was evaporated at a temperature of 55-60°C under reduced pressure to a yellow-brown oil, which was dissolved in water (60 ml) and filtered from a little insoluble matter. To the filtrate water (105 ml) was added and mixed-bed exchange resin MB-150 (Sigma # A-5710; 90 g) was added and stirred for a period of 10 minutes. The resin was removed by filtration and washed with water.

The yellow-orange filtrate was cautiously concentrated by rotary evaporation at 55-60°C under reduced pressure to a volume of 5-10 ml. Upon cooling to ambient temperature colorless crystals of the product appeared spontaneously. The suspension was stirred for half an hour at the same temperature and subsequently stored overnight at 5°C. The product was collected by filtration and washed once with ice-water (4 ml) and once with acetone (6 ml) and finally dried in vacuum to give 4.38 g (43 %) of a white, crystalline powder.
m.p. 259-260°C (dec.).
Analysis calcd. for C₁₅H₂₁N₂O₅P (MW= 340.31) C 52.94, H 6.22, N 8.23, P 9.10; Found (calcd. on dry matter; water content 11.6 %): C 52.81, H 6.35, N 8.18, P 8.99;
¹H-NMR (400 MHz; DMSO-d₆), δ (ppm): 8.10 (broad d; 1H), 7.57 (m, 1H), 7.33 (m; 3H), 4.36 (m, 2H), 3.58 (t, 2H, J_{P,H}= 4.5 Hz), 3.08 (s, 9H), 2.51 (s, 3H).
¹³C-NMR (400 MHz; DMSO-d₆), δ (ppm): 171.7, 135.7 (d, J_{P,C}= 7.7 Hz), 132.5, 126.0, 124.2 (d, J_{P,C}= 5.2 Hz), 123.8, 117.4, 115.8, 112.2 (very small d) , 65.6 (m), 59.9 (d, J_{P,C}= 5.2 Hz), 53.6 (t, J_{N,C}= 3.5 Hz), 22.9.

### Step 2: Preparation of 3-Indoxyl choline phosphate (V)

A 2 N solution of ammonia in methanol (28 ml) was added at a temperature of 10-20°C to 1-Acetyl-3-indoxyl choline phosphate (2.72 g; 8 mMol) under nitrogen. The educt quickly dissolved leaving a slightly blue solution. After a period of 3 hours acetone (80 ml) was added. The solution was seeded whereby a white, crystalline precipitate soon appeared. The suspension was stirred for half an hour at ambient temperature and subsequently for two hours in an ice bath. The product was collected by filtration through a glass filter funnel and the crystals washed with acetone (2 x 20 ml). The product was dried in vacuo leaving 1.58 g (66 %) of a white, crystalline powder.
m.p. 261-262°C (dec.).
Analysis calcd. for C₁₃H₁₉N₂O₄P (MW= 298.28) : C 52.35, H 6.42, N 9.39, P 10.38; Found (calcd. on dry matter; water content 0.6 %) : C 52.42, H 6.55, N 9.45, P 10.26;
¹H-NMR (400 MHz; D₂O), δ (ppm): 7.52 (d; 1H), 7.30 (d, 1H), 7.08 (td; 1H), 7.06 (d, 1H), 7.00 (td, 1H), 4.11 (m, 2H), 3.27 (t, 2H, J_{P,H}= 4.25 Hz), 2.75 (s, 9H) ;
¹³C-NMR (400 MHz; D₂O), δ (ppm): 132.7, 129.7 (d, J_{P,C}= 7.8 Hz), 122.0, 119.3 (d, J_{P,C}= 4.4 Hz), 119.0, 116.6, 112.5, 111.6, 65.3 (m), 59.6 (d, J_{P,C}= 5.0 Hz), 53.1.
UV (0.1 N HEPES/NaOH pH 7.0) : λₘₐₓ = 280 nm, ε = 5020 L Mol⁻¹ cm⁻¹.

### Example 3: Evaluation of the proper and preferred additive or combination of cleavage rate enhancers for Clostridium perfringens PC-PLC

To evaluate the proper and preferred additive or combination of additives for rapid color formation from X-phos-choline (X-CP) by enzymatic hydrolysis with PC-PLC of *Clostridium perfringens,* simple test tube experiments were conducted.

Preliminary tests at ≈ 40°C showed that a weak enhancement of the cleavage rate of the enzyme is obtained by using Octyl-β-D-thiogalactopyranoside (Biosynth O-2700) at 0.1 %; Octyl-β-D-thioglucopyranoside (Biosynth O-2710) at 0.1 %; N-Nonanoyl-N-methyl-glucamin (Fluka 74315) in the range 0.1 % to 1%; and Glycocholic acid sodium salt (Sigma G-7132) at 0.1 %, to X-CP (the values given in % indicate the concentrations of the additives in weight per volume). For all these detergents and surfactants, respectively, no additional enhancement was observed when combined with BSA.

Further experiments were conducted at 60°C, which is near the temperature optimum of the enzyme, cf. example 4. The basic parameters were as follows:
≈ 1,7 mg of X-CP were dissolved in 1 ml of 0.1 N HEPES/NaOH-buffer of pH 7.0 giving a ≈ 4 mM solution of the substrate.

To probe the acceleration of the enzymatic cleavage, the following chemicals were added to the buffer, either alone or in combination:
- Bovine Serum Albumin (Fluka 05470) at 0.1 % (BSA)
- Magnesium sulfate heptahydrate (Fluka 63140) at 0.01 % (Mg²⁺)
- Calcium chloride dihydrate (Fluka 21098) at 0.01 % (Ca²⁺)
- Manganese(II)sulfate monohydrate (Fluka 63554) at 0.01 % (Mn²⁺)
- Cobalt (II) sulfate heptahydrate (Fluka 00622) at 0.01 % (Co²⁺)
- Nickel(II)sulfate hexahydrate (Fluka 72280) at 0.01 % (Ni²⁺)
- Zinc sulfate heptahydrate (Fluka 96500) at 0.01 % (Zn²⁺)

To each solution was added 1 Unit of Phospholipase C from *Clostridium perfringens* (Sigma P-4030, cf. examples 4-6 below). The tubes were checked visually after defined periods of time. Table 1 shows the color of the solution using various combinations of the above reagents after 1 hour at 60°C.

**Table 1**

| Combination | No Additive | Mg²⁺ | Ca²⁺ | Mn²⁺ | Co²⁺ | Ni²⁺ | Zn²⁺ |
|---|---|---|---|---|---|---|---|
| No Additive | Bluish | Bluish | Colorless | Pale blue | Light blue | Pale blue | Colorless |
| BSA | Bluish | Bluish | Colorless | Light blue | **Deep dark blue** | Colorless | Colorless* |

| | | | | | | | |
|---|---|---|---|---|---|---|---|
| * Precipitation of Zinc hydroxide! | | | | | | | |

Table 1 clearly shows the drastic effect of additives for the cleavage rate. So, the addition of BSA and Co²⁺ to a substrate comprising X-CP strongly accelerate the cleavage rate for *Clostridium perfringens* PC-PLC. The enhancement with the combination of BSA and cobalt ions is of such an outstanding order to make X-phos-choline (X-CP) a very useful compound and substrate, respectively, for detecting PC-PLC of *Clostridium perfringens.*

Remarkable variations in the rate enhancement are, however, observed requiring a proper selection of the additive or the combination of additives to obtain a significant rate enhancement. As compared to BSA/Co²⁺ system the addition of manganese or nickel ions only slightly accelerates the cleavage rate whereas calcium and zinc ions inhibit. In combination with BSA, manganese ions weakly enhance whereas nickel ions inhibit. As will become apparent, the preferred selection of additive(s) for a cleavage rate enhancement may vary for each particular microorganism and, thus, may need to be addressed for each particular microorganism.

### Examples 4-6: Colorimetric assay of PC-PLC from Clostridium perfringens using X-phos-choline

In these examples a Pye Unicam SP 6-450 UV/VIS Spectrophotometer was used for the experiments. The spectrometer was set to 652 nm.

The general procedure for the studies in Example 5 and 6 was as follows:

All components were dissolved in 0.1 M HEPES/NaOH-buffer of pH 7.0. Bovine Serum Albumin (BSA; Fluka 05470) and different amounts of salts of cobalt were dissolved in 2 ml of the buffer solution in a cuvette. An aliquot from a stock solution of PC-PLC from *Clostridium perfringens* (Sigma P-4039: Type XIV, chromatographically purified, lyophilized powder in buffered salts; Activity: 300 Units per mg protein (Lowry) using egg-yolk phosphatidylcholine) was added. The stock solution of the enzyme was prepared by dissolving the powder in the above-mentioned buffer to give a final concentration of 1 Unit / 10 µL solution. The mixture in each cuvette was incubated at 60°C for half an hour.

In the meantime X-phos-choline (X-CP) was dissolved in 1 ml of the buffer and this solution was transferred to the cuvette to start the enzymatic hydrolysis of X-CP and the substrate, respectively. The photometer readings were noted after defined periods of time for various temperatures as well as for various concentrations of the compound and substrate, respectively, as well as of the enzyme.

### Example 4:

### Effect of Temperature

In a first set of experiments the influence of temperature on the enzymatic cleavage of X-CP by PC-PLC from *Clostridium perfringens* was investigated. In this case the cuvettes were not incubated prior to the addition of X-CP and the substrate, respectively, but the reaction was started by addition of the enzyme:

To a solution of X-CP in the above mentioned buffer, BSA and Cobalt(II)chloride hexahydrate (Fluka 60820) were added to give the following final concentrations (the values given in % indicate the concentrations in weight per volume; in a volumina of 3 ml):
X-CP at 20 mM (24.7 mg / 3 ml), BSA at 0.3 %, Cobalt(II)chloride hexahydrate at 0.025 % (1.05 mM).
The cuvettes were incubated at ambient temperature (22°C) as well as at 41°C, 46°C, 51°C, 55°C and 60°C, respectively. Each measurement was started by adding 2 Units of PC-PLC (Sigma P-4039, see above) to the cuvette.

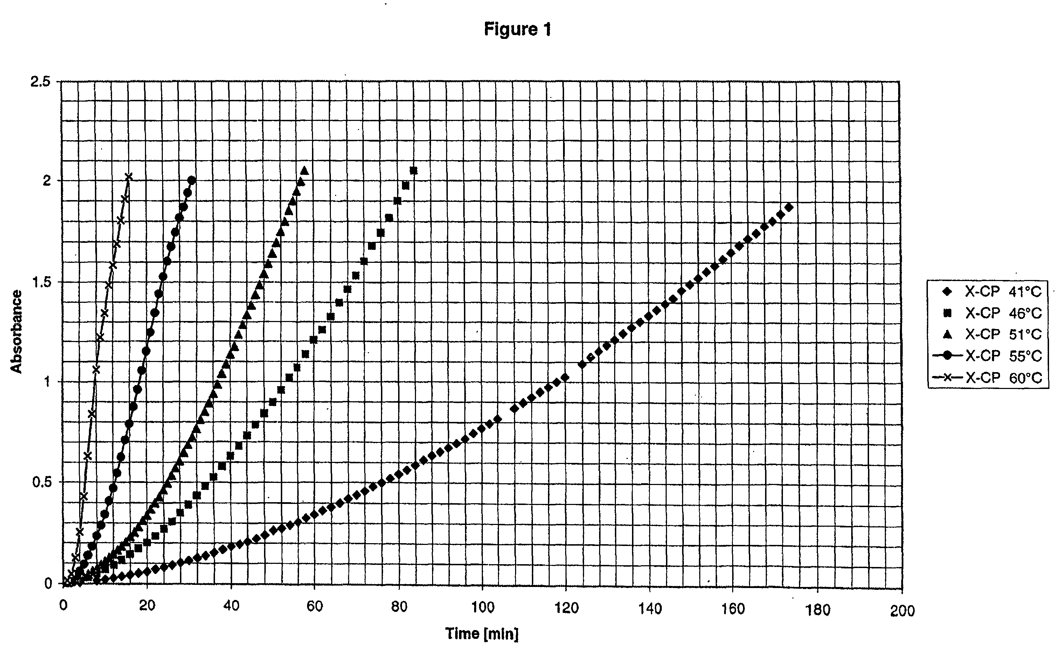

Figure 1 shows the change of the absorbance A as a function of time for various temperatures.

Reaction rates Δc / Δt (c = concentration) of the enzymatic cleavage were calculated from the slopes ΔA / Δt of the linear part of each curve using the equation of Lambert-Beer: ΔA = ε • Δc • d (d = 1 cm; absorption coefficient ε = 6000 L mol⁻¹ cm⁻¹ for the 5,5'-Dibromo-4,4'-dichloro-indigo dye).

Specific activities were calculated from the rates in consideration of the amount of enzyme (2 Units = 6.667 µg) used.

Table 2 lists reaction rates (related to a volume of 3 ml) and specific activities [1 nMol min⁻¹ (µg protein)⁻¹ = 1 µMol min⁻¹ (mg protein)⁻¹] of *Clostridium perfringens* PC-PLC for the temperatures investigated.

**Table 2**

| Temperature | Rate | Specific Activity |
|---|---|---|
| [°C] | [nMol/min] | [µMol min⁻¹ mg⁻¹] |
| 22 | 0.65 | 0.1 |
| 41 | 8.1 | 1.2 |
| 46 | 19.7 | 3.0 |
| 51 | 25.8 | 3.9 |
| 55 | 48.5 | 7.3 |
| 60 | 106.8 | 16.0 |

Table 2 shows the marked influence of temperature on enzyme activity. For every 10°C of rise in temperature the enzymatic activity increased by a factor of 3-4. For example, at 60°C the cleavage is approximately 160 times faster as at ambient temperature.

### Example 5:

### Dependence upon Concentration of X-CP

In this second set of experiments, which were conducted at a temperature of 58-59°C, the influence of the concentration of X-CP was studied. The fix parameters were as follows: BSA at 0.1 %, Cobalt(II)sulfate heptahydrate (Fluka 00622) at 0.01 % (0.356 mM), 1 Unit of enzyme, cf. example 4.

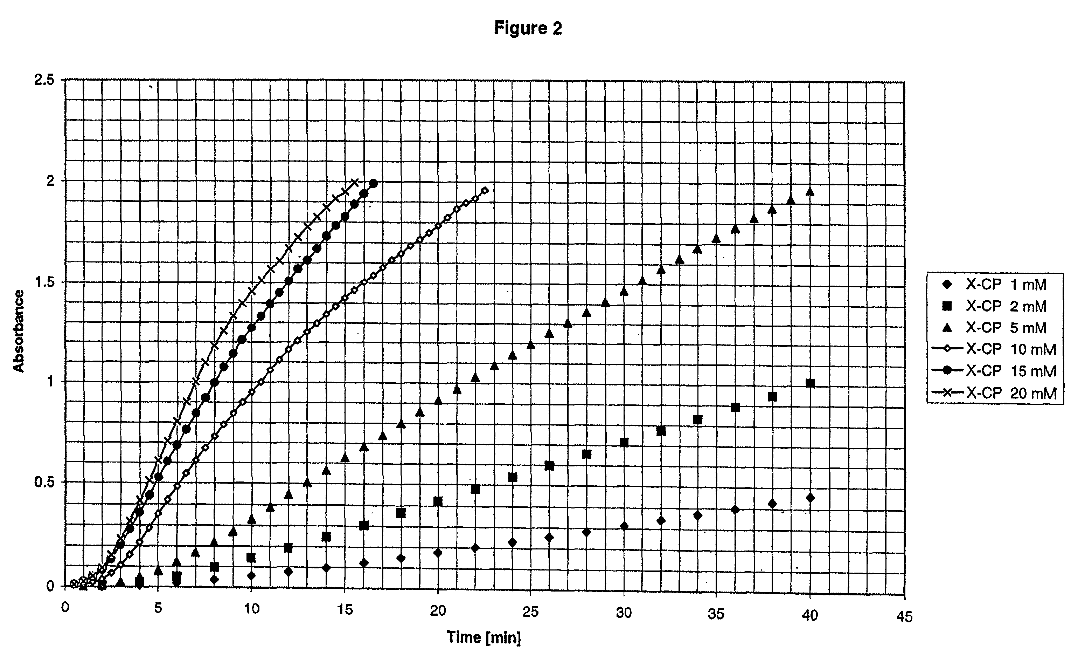

Figure 2 shows the time course of the increase of the absorbance at 652 nm for the concentrations of X-CP used. Table 3 shows rates of cleavage (again for 3 ml, which is the volume in the cuvettes) and specific activities calculated as described in example 4.

**Table 3**

| X-CP Concentration | Rate | Specific Activity |
|---|---|---|
| [mM] | [nMol/min] | [µMol min⁻¹ mg⁻¹] |
| 1 | 7.2 | 2.15 |
| 2 | 14.7 | 4.4 |
| 5 | 30.0 | 9.0 |
| 10 | 64.1 | 19.2 |
| 15 | 83.3 | 25.0 |
| 20 | 100.8 | 30.25 |

PC-PLC activity was not entirely proportional to the X-CP concentration. For the higher concentrations of 15 mM and 20 mM the activity decreased thereby pointing to a Michaelis behavior of the PC-PLC activity. Similar behavior has been observed with the natural substrate Phosphatidylcholine in a Phospholipase C-Alkaline Phosphatase coupled assay (cf. E.L. Krug, C. Kent; Archives of Biochemistry and Biophysics 231 (1984), p. 406).

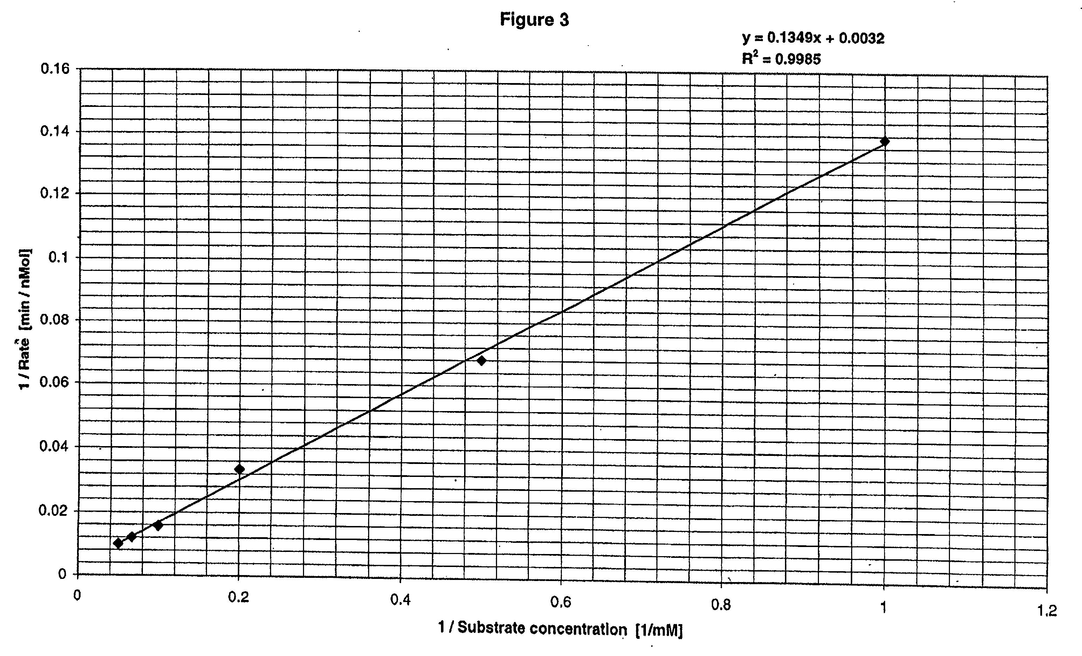

Thus kinetic parameters could be determined from a Lineweaver-Burk plot of I/Rate versus 1/Substrate concentration, cf. Figure 3. From the linear regression analysis of the data, values for the rate of cleavage under saturating conditions vₘₐₓ and for the Michaelis-Menten constant Kₘ could be estimated:
vₘₐₓ = 1 / 0.0032 = 312 nMol min⁻¹ (for 1 Unit of enzyme used = 3.333 µg).
vₘₐₓ = 312 nMol min⁻¹ (3.333 µg enzyme)⁻¹ = 93.6 nMol min⁻¹ (µg enzyme)⁻¹ ≈ 94 µMol min⁻¹ (mg enzyme)⁻¹.
Kₘ = 0.1349 / 0.0032 ≈ 42 mM.

This rough estimation of Kₘ shows a rather low affinity of the enzyme for X-CP. However, from the value of vₘₐₓ it is obvious that turnover of enzyme-bound X-CP is high enough to be useful in conducting enzyme assays, cf. example 6.

### Example 6

### Dependence upon Enzyme Concentration

These experiments were conducted to find out about the' correlation between the cleavage rates and the amount of enzyme applied. The parameters were as follows:
X-CP at 10 mM (12.35 mg / 3 ml), BSA at 0.1 %, Cobalt(II)sulfate heptahydrate at 0.01 %, cf. example 5.
All the cuvettes were incubated at 58-59°C.

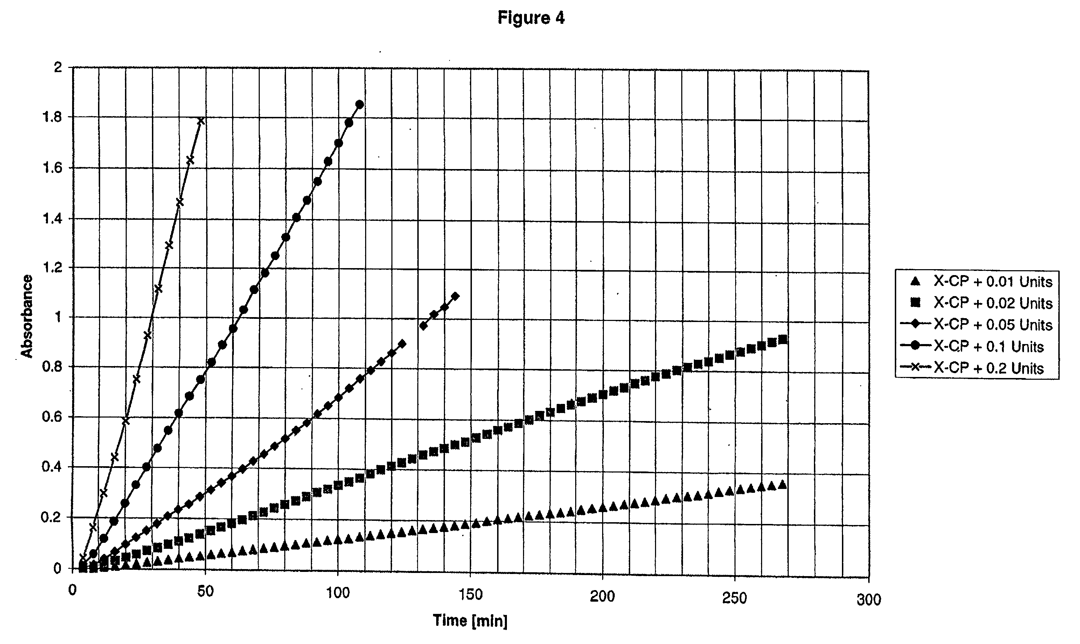

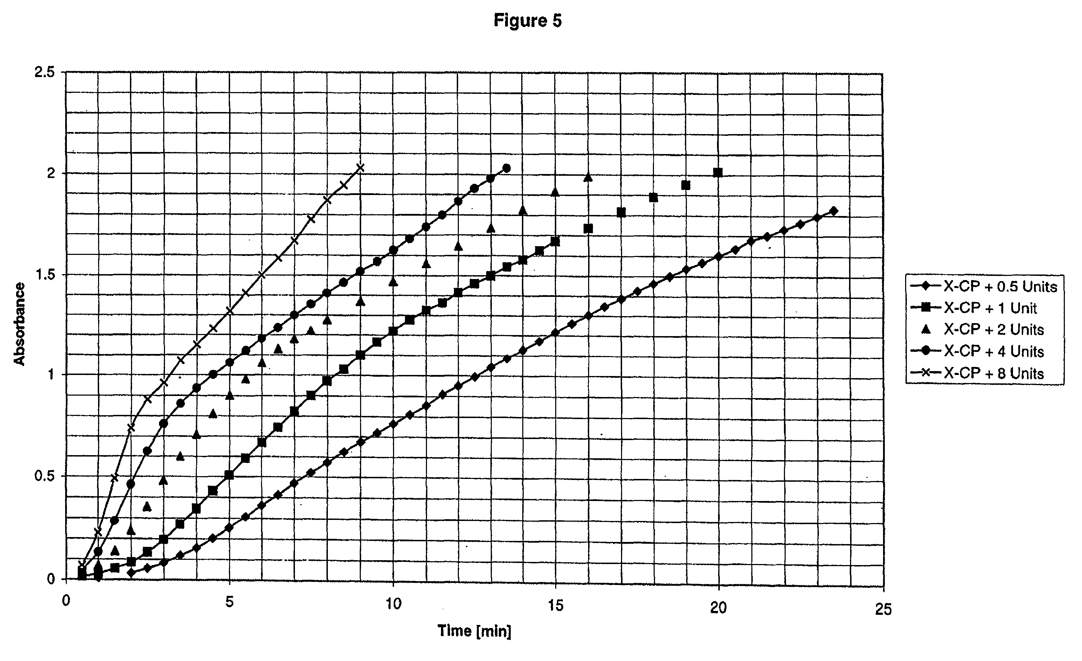

Figures 4 and 5 show the time course of X-CP hydrolysis using 0.01 to 0.2 and 0.5 to 8 Units of PC-PLC from *Clostridium perfringens,* respectively.

Again, turnover rates were calculated from the slopes of the linear part of the curves as described in example 4. Table 4 shows rates of cleavage and specific activities for various amounts of enzyme used (1 Unit = 3.33 µg enzyme, cf. the general procedure above).

**Table 4**

| Enzyme | | ^{*1,6*}√ *Enzyme* | Rate | Specific Activity | CF |
|---|---|---|---|---|---|
| [Units] | [µg] | ^{*1,6*}√ *[µg]* | [nMol/min] | [µMol min⁻¹ mg⁻¹] | *[µMol min*^{*-1 1,6*}√ *[mg]* ^{*-1*}*]* |
| 0.01 | 0.0333 | *0.1193* | 0.74 | 22.2 | *6.2* |
| 0.02 | 0.0666 | *0.1841* | 1.9 | 28.2 | *10.2* |
| 0.05 | 0.1666 | *0.3263* | 4.7 | 28.4 | *14.5* |
| 0.1 | 0.3333 | *0.5033* | 9.4 | 28.2 | *18.7* |
| 0.2 | 0.6666 | *0.7761* | 22.0 | 33.0 | *28.3* |
| 0.5 | 1.6666 | *1.376* | 52.1 | 31.25 | *37.85* |
| 1 | 3.3333 | *2.122* | 78.6 | 24.0 | *37.05* |
| 2 | 6.6666 | *3.273* | 120.2 | 18.2 | *36.7* |
| 4 | 13.333 | *5.048* | 173.6 | 13.0 | *34.4* |
| 8 | 26.666 | *7.785* | 260.4 | 9.8 | *33.45* |

From Table 4 one recognizes an almost linear relationship between the rates and the amount of enzyme present in the range of 0.02 to 0.5 Units while the specific activity is reduced for 0.01 Units and declines substantially for the higher concentrations of enzyme (≥ 1 Unit).

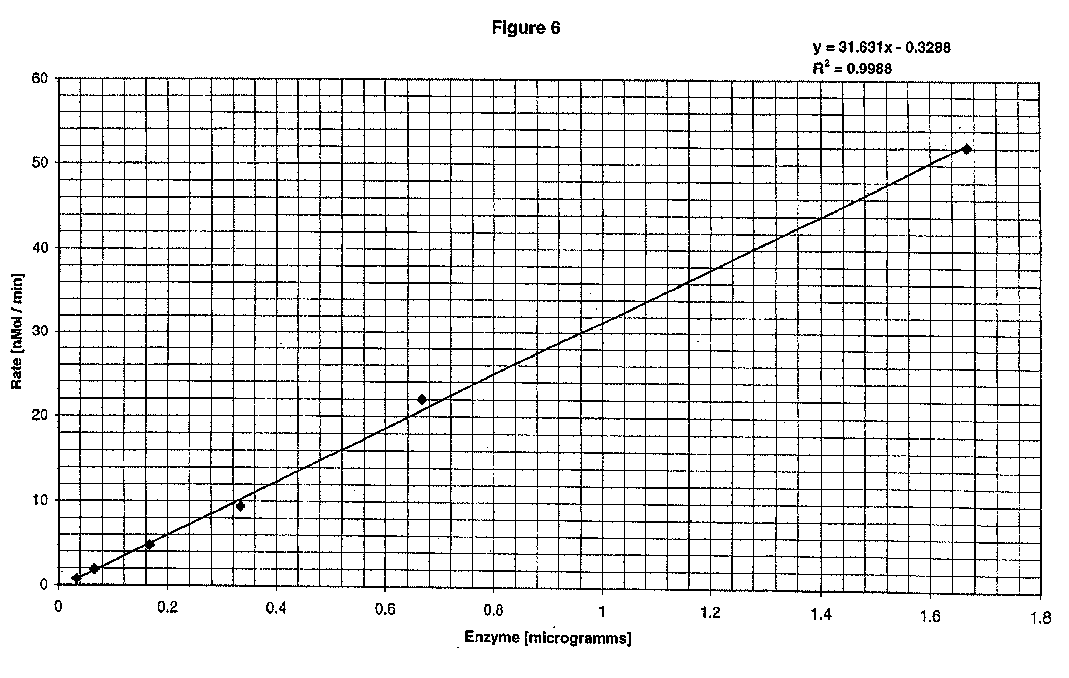

Figure 6 is a plot of the rates versus amount of enzyme in the range 0.01 to 0.5 Units (33 ng to 1667 ng). The proportionality between the rate and the amount of enzyme applied can be clearly seen.

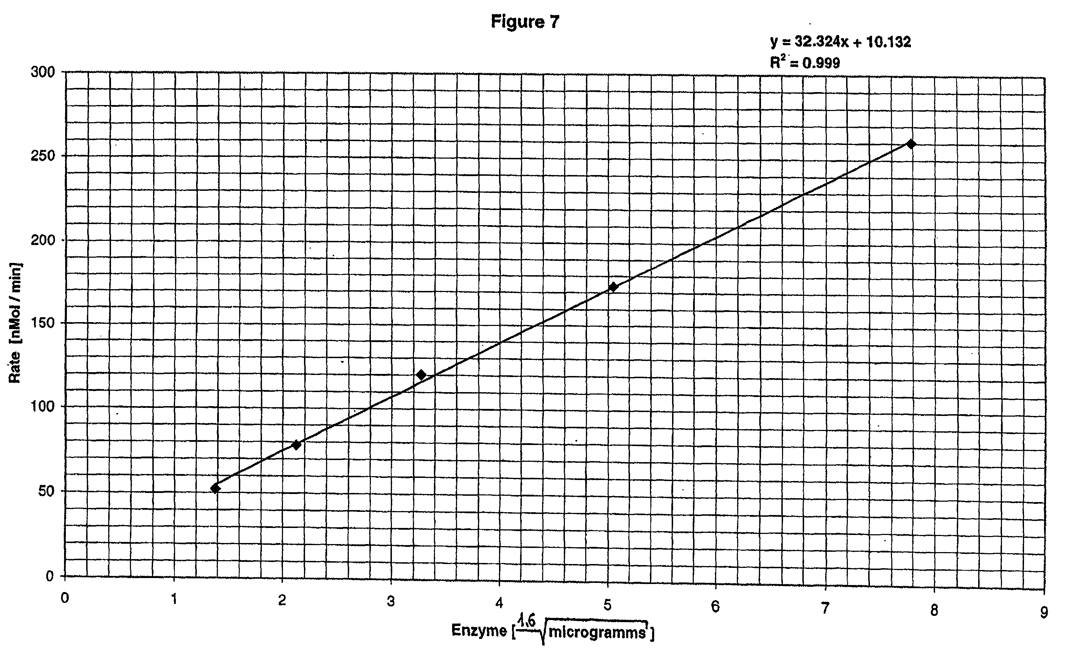

In the range 0.5 to 8 Units of enzyme where there is no linearity any more, the rate is, however, proportional to the expression ^{1,6}√ amount of Enzyme; the factor "CF" in Table 4 is approximately constant. Figure 7 shows this linear relationship between the rates and ^{1,6}√ amount of Enzyme in the range 0.5 to 8 Units (1.66 to 27 µg) of enzyme present. Thus using a substrate comprising X-CP the PC-PLC enzyme of *Clostridium perfringens* can be assayed quite accurately at least in a range from 33 ng to 27 µg. The example shows that this assay already works under simple, non-optimized conditions.

### Example 7: Preparation and Inoculation of plating media for testing the efficacy of X-CP and Y-CP

### MICROBIAL STRAINS

*Bacillus cereus* (ATCC 13061), *Bacillus thuringiensis* (ATCC 33680), and *Pseudomonas aeruginosa* (ATCC 15442) were stored at 4°C on Difco Brain Heart Infusion Agar (BHIA) slants. Cultures were transferred to a fresh BHIA slant monthly. A loopful of *B. cereus, B. thuringiensis*, and *P. aeruginosa* cells was aseptically transferred to Difco Brain Heart Infusion Broth (BHIB) and incubated at 35°C for 24 hours. After incubation, these cells were used to streak the test plating media for the colorimetric detection of PC-PLCs.

### PREPARATION OF THE PLATING MEDIA CONTAINING THE CHROMOGENIC COMPOUNDS

Difco Tryptic Soy Agar (TSA) was used as the basal plating medium. TSA was prepared according to manufacturers' instructions. Boiling the medium melted the agar in order to divide the medium. To the hot medium the following chemicals and corresponding concentrations were added:
Manganese chloride tetrahydrate (Sigma M-3634) at 0.1 % (wt/vol). Tween 80 (Polyoxyethylene sorbitan monooleate; Difco) at 0.05 % (wt/vol).
Sorbitol (Difco 0179) at 20 % (wt/vol).
Cupric sulfate pentahydrate (Sigma C-7631) at 0.078 g/100 ml.
Zinc sulfate heptahydrate (Sigma Z-4750) at 0.015 g/100 ml.
Calcium chloride dihydrate (Fisher) at 0.074 g /100 ml.
Magnesium sulfate anhydrous (Sigma M-7506) at 0.012 g/100 ml.

The TSA media containing the above chemicals in various combinations were autoclaved at 121°C for 15 minutes. After autoclaving the media were placed in a water bath set at a temperature of 50°C.

Bovine Serum Albumin (BSA; Serologicals 82-067) and X-phos-choline (X-CP) or Y-phos-choline (Y-CP) were dissolved in deionized water. The solution was filter sterilized through a 0.45 µm filter. An appropriate volume of the filtrate was aseptically added to the various TSA media that were previously cooled to 50°C.

The final concentration of the BSA was 320 mg/100 ml. The final concentration of X-phos-choline (X-CP) or Y-phos-choline (Y-CP) was 32 mg/100 ml. The complete TSA media were poured into Petri plates and allowed to solidify. The plates were held at room temperature overnight in the dark to surface dry the media.

### INOCULATION OF THE TSA MEDIA TESTING THE EFFICACY OF X-CP AND Y-CP

Cells from the various 24-hour BHIB cultures mentioned above were streaked on the TSA media containing the various chemical combinations. The plates inoculated with *Bacillus cereus, Bacillus thuringiensis* and *Pseudomonas aeruginosa* were incubated aerobically at 35°C for 24 hours. After incubation, the colonial morphologies were determined with special emphasis on coloration.

### RESULTS

The colonial morphologies, including coloration, for *Bacillus cereus, Bacillus thuringiensis* and *Pseudomonas aeruginosa* on the various TSA media are presented in Table 5.

**Table 5**

| **Test Variables** TSA + Additives: | ***Bacillus cereus*** | ***Bacillus thuringiensis*** | ***Pseudomonas aeruginosa*** |
|---|---|---|---|
| No Additives | White | White | Off white |
| X-CP + BSA | Light to medium Turquoise color | White | Off white |
| X-CP + BSA + Cupric sulfate | Faint Turquoise color | White | Off white |
| X-CP + BSA + Sorbitol | Very faint Turquoise color | White | Off white |
| X-CP + BSA + Tween 80 | **Dark Turquoise color** | White | Light Turquoise |
| X-CP + BSA + Tween 80 + Magnesium sulfate | Medium to dark Turquoise color | White | Light to medium Turquoise |
| X-CP + BSA + Tween 80 + Calcium chloride | Medium Turquoise color | White | Yellowish Turquoise |
| X-CP + BSA + Tween 80 + Zinc sulfate | Medium Turquoise color | White | **Medium to dark Turquoise** |
| X-CP + BSA + Tween 80 + Manganese chloride | **Dark intense Blue color** | **Medium Turquoise color** | Yellowish Turquoise |

The colonial morphologies, including coloration, for *Bacillus cereus, Bacillus thuringiensis* and *Pseudomonas aeruginosa* on the various TSA media with a substrate comprising Y-CP are presented in Table 6.

**Table 6**

| **Test Variables** TSA + Additives: | ***Bacillus cereus*** | ***Bacillus thuringiensis*** | ***Pseudomonas aeruginosa*** |
|---|---|---|---|
| No Additives | White | White | Off white |
| Y-CP + BSA | Faint Turquoise color | White | Off white |
| Y-CP + BSA + Cupric sulfate | White | White | Off white |
| Y-CP + BSA + Sorbitol | White | White | Off white |
| Y-CP + BSA + Tween 80 | **Medium Turquoise color** | White | Very light Turquoise |
| Y-CP + BSA + Tween 80 + Magnesium sulfate | Medium Turquoise color | White | Light Turquoise |
| Y-CP + BSA + Tween 80 + Calcium chloride | Faint Turquoise color | White | Yellowish very light Turquoise |
| Y-CP + BSA + Tween 80 + Zinc sulfate | Light Turquoise color | White | **Medium Turquoise** |
| Y-CP + BSA + Tween 80 + Manganese chloride | **Dark Blue color** | **Medium Turquoise color** | Yellowish Turquoise |

As can be seen from Table 5 and 6, the nonionic detergent and surfactant, respectively, Tween 80 caused an enhancement of the expression of the PC-PLC enzyme as indicated by the turquoise to blue colony color for *Bacillus cereus.* The further addition of manganese chloride led to a turquoise color for the *Bacillus thuringiensis* colonies, whereas zinc sulfate caused an increase of the expression of PC-PLC for *Pseudomonas aeruginosa as* indicated by medium to dark turquoise colony color.

The data in Table 5 and 6 clearly show that the use of the chromogenic compounds X-phos-choline (X-CP) and Y-phos-choline (Y-CP) allow the detection of PC-PLC by formation of a turquoise to blue colony color. The two compounds and substrates, respectively, behave quite similarly, however, the color intensity with Y-CP is slightly lesser. These data also show that the expressions of PC-PLC by the tested bacteria are influenced by the particular additive or combination of additives and their concentrations being added to the plating medium.

It should be noted that while the above examples are concerned with 3-Indoxyl choline phosphate and with X-phos-choline, the preferred compounds of formula IV, it is apparent from the general disclosure above that similar results will be obtained with other substrates of formula I if the substituents R¹, R², R³, R⁴ of the benzene nucleus of formula I compounds are selected in a manner known, per se, in the chemistry of indigo-type dyes and in histochemistry.

Although certain presently preferred embodiments of the present invention have been specifically described herein, it will be apparent to those skilled in the art to which the invention pertains that variations and modifications of the various embodiments shown and described herein may be made without departing from the spirit and scope of the invention. Accordingly, it is intended that the invention be limited only to the extent required by the appended claims and the applicable rules of law.

Generally, the invention provides for safe and sensitive detection of potentially pathogenic bacterial activity of such microbes as *Clostridium perfringens, Bacillus cereus, Pseudomonas aeruginosa, Listeria monocytogenes, Helicobacter pylori, Legionella pneumophila* and others in potentially infected materials including physiological samples or consumable goods such as foods and beverages.

## Claims

1. A chromogenic 3-Indoxyl choline phosphate compound of formula I: 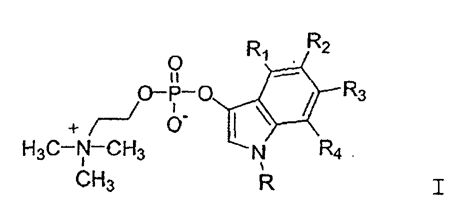 wherein R is selected from the group consisting of hydrogen and C₁₋₄ alkyl, such as methyl, ethyl, propyl and butyl while R¹, R², R³, and R⁴ are independently selected from the group consisting of hydrogen, halogen, cyano, nitro, carboxy, amino, amino substituted with one or two C₁₋₄ alkylgroups, aminomethyl, hydroxy, C₁₋₄ alkoxy, carboxyalkyl, and sulphonyl.

2. The compound of claim 1, wherein R is selected from the group consisting of hydrogen and methyl; R¹ is selected from the group consisting of hydrogen and halogen;. R² is selected from the group consisting of hydrogen, halogen, cyano, nitro; and R³ and R⁴ are independently selected from the group consisting of hydrogen and halogen.

3. The compound of claim 1 or 2, wherein R is hydrogen, R¹ is selected from hydrogen and chlorine, R² is selected from hydrogen and bromine, R³ is selected from hydrogen, chlorine and fluorine, and R⁴ is hydrogen; and preferably wherein R, R¹ and R⁴ is hydrogen, R² is hydrogen or bromine, and R³ is chlorine.

4. 5-Bromo-4-chloro-3-indoxyl choline phosphate represented by formula (IV) :

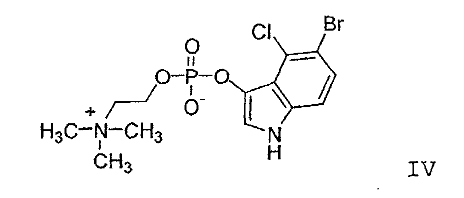

5. 3-Indoxyl choline phosphate represented by formula (V):

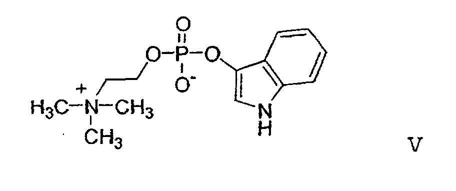

6. A substrate for detecting a Phosphatidylcholine-specific Phospholipase C enzyme as an indication of microbial activity; said substrate comprising at least one chromogenic 3-Indoxyl choline phosphate compound as defined in any of claims 1-5.

7. The substrate of claim 6 additionally comprising at least one additive selected from the group consisting of serum albumin, surfactants and metal ions.

8. The substrate of claim 6 additionally comprising at least one metal ion and at least one member selected from the group consisting of surfactants and serum albumin.

9. The substrate of claim 7 or 8, wherein said metal ion is a divalent ion of cobalt, manganese, nickel, zinc, calcium or magnesium.

10. A method of detecting a Phosphatidylcholine-specific Phospholipase C enzyme comprising the steps of:
contacting a sample suspected of containing said phosphatidylcholine-specific Phospholipase C enzyme with a substrate containing at least one 3-Indoxyl choline phosphate compound of formula I: 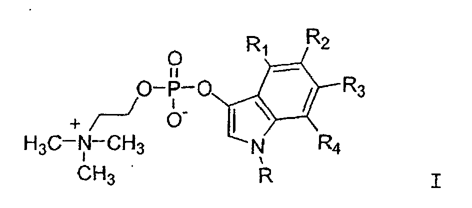 wherein R is selected from the group consisting of hydrogen and C₁₋₄ alkyl, such as methyl, ethyl, propyl and butyl while R¹, R², R³, and R⁴ are independently selected from the group consisting of hydrogen, halogen, cyano, nitro, carboxy, amino, amino substituted with one or two C₁₋₄ alkylgroups, aminomethyl, hydroxy, C₁₋₄ alkoxy, carboxyalkyl, and sulphonyl; and wherein said compound being susceptible to cleavage by said enzyme yielding a dye; and
monitoring for color formation as a consequence of said sample suspected of said Phosphatidylcholine-specific Phospholipase C enzyme.

11. The method of claim 10, wherein R is selected from the group consisting of hydrogen and methyl; R¹ is selected from the group consisting of hydrogen and halogen; R² is selected from the group consisting of hydrogen, halogen, cyano, nitro; and R³ and R⁴ are independently selected from the group consisting of hydrogen and halogen.

12. The method of claim 10 or 11, wherein R is hydrogen, R¹ is selected from hydrogen and chlorine, R² is selected from hydrogen and bromine, R³ is selected from hydrogen, chlorine and fluorine, and R⁴ is hydrogen; and preferably wherein R, R¹ and R⁴ is hydrogen, R² is hydrogen or bromine, and R³ is chlorine.

13. The method of claim 10, wherein said at least one compound is 5-Bromo-4-chloro-3-indoxyl choline phosphate represented by formula IV:

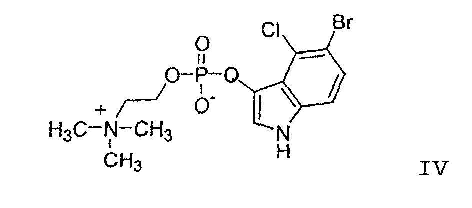

14. The method of any of claims 10 to 13, wherein said substrate further comprises at least one additive selected from the group consisting of serum albumin, surfactants and metal ions.

15. The method of any of claims 10 to 13, wherein said substrate further comprises at least one metal ion and at least one member selected from the group consisting of surfactants and serum albumin.

16. The method of claim 14 or 15, wherein said metal ion is a divalent ion of cobalt, manganese, nickel, zinc, calcium or magnesium.

17. The method of any of claims 10 to 16, wherein said Phosphatidylcholine-specific Phospholipase C enzyme derives from a pathogen selected from the group consisting of *Clostridium perfringens, Clostridium novyi, Bacillus cereus, Bacillus thuringiensis, Pseudomonas aeruginosa, Helicobacter pylori, Legionella pneumophila, Listeria monocytogenes* and *Candida albicans.*

18. A method of detecting microbial activity in a sample; said method comprising the steps of (i) combining said sample with a substrate comprising at least one compound of formula I as defined in any of claims 1 to 5; and (ii) inspecting said sample combined with said substrate by colorimetric means.

19. The method of claim 18, wherein said substrate further comprises at least one additive selected from the group consisting of serum albumin, surfactants and metal ions, preferably said metal ion is a divalent ion of cobalt, manganese, nickel, zinc, calcium or magnesium.

20. The method of claim 18 or 19 for use as a screening test in the detection of bacteria of the group including *Clostridium perfringens, Clostridium novyi, Bacillus cereus, Bacillus thuringiensis, Pseudomonas aeruginosa, Helicobacter pylori, Legionella pneumophila* and *Listeria monocytogenes* as well as in the detection of the yeast *Candida albicans.*

21. A kit for detecting a Phosphatidylcholine-specific Phospholipase C enzyme as an indication of microbial activity; said kit including at least one compound of formula I as defined in any of claims 1 to 5.

22. An assay method for detection of PC-PLC by means of a substrate as defined in any of claims 6 to 9.

23. An assay method for detection of the presence of pathogenic strains of *Clostridium perfringens* in a sample suspected to contain such strains by means of a substrate as defined in any of claims 6 to 9.

24. An assay method for detection of the presence of pathogenic strains of *Clostridium perfringens* in a sample suspected to contain such strains by means of a substrate comprising 5-Bromo-4-chloro-3-indoxyl choline phosphate represented by formula (IV) or 3-Indoxyl choline phosphate represented by formula (V), bovine serum albumin and at least one source of Co²⁺ ions.

25. An assay method for detection of the presence of pathogenic strains of *Bacillus cereus* or *Bacillus thuringiensis* in a sample suspected to contain such strains by means of a substrate comprising 5-Bromo-4-chloro-3-indoxyl choline phosphate represented by formula (IV) or 3-Indoxyl choline phosphate represented by formula (V), bovine serum albumin, polyoxyethylene sorbitan monooleate and at least one source of Mn²⁺ ions.

26. An assay method for detection of the presence of pathogenic strains of *Pseudomonas aeruginosa* in a sample suspected to contain such strains by means of a substrate comprising 5-Bromo-4-chloro-3-indoxyl choline phosphate represented by formula (IV) or 3-Indoxyl choline phosphate represented by formula (V), bovine serum albumin, polyoxyethylene sorbitan monooleate and at least one source of Zn²⁺ ions.

27. A method of identifying a bacterial microorganism of interest, which is capable of producing a phosphatidylcholine-specific phospholipase C (PC-PLC) enzyme comprising the steps of:
(A) providing a test sample suspected of containing said microorganism of interest;
(B) preferably submitting said test sample to an enrichment broth step;
(C) transferring a portion, at least, of said test sample or the product obtained in step (B) to a medium suitable for culturing said microorganism; said medium containing at least one compound of formula I capable of producing a color when exposed to said microorganism;
(D) cultivating said medium with said transferred portion for developing at least one colony exhibiting said color; and
(E) recovering a portion, at least, of said colored colony for final identification.

28. A method of preparing a chromogenic 3-Indoxyl choline phosphate compound of formula I 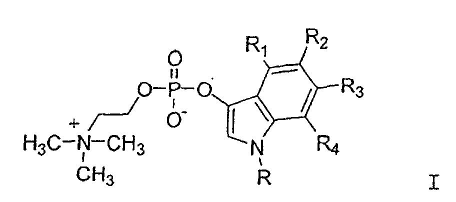 wherein R is selected from the group consisting of hydrogen and C₁₋₄ alkyl, such as methyl, ethyl, propyl and butyl while R¹, R², R³, and R⁴ are independently selected from the group consisting of hydrogen, halogen, cyano, nitro, carboxy, amino, amino substituted with one or two C₁₋₄ alkylgroups, aminomethyl, hydroxy, C₁₋₄ alkoxy, carboxyalkyl, and sulphonyl;
comprising the steps of reacting a corresponding Indoxyl-3-dichlorophosphate of formula II with a salt of choline in order to obtain an intermediate compound III according to the reaction: 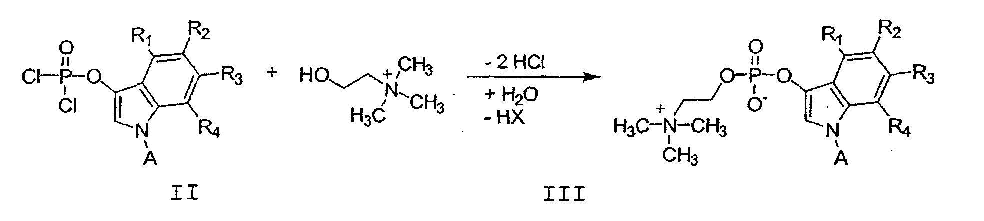 in which A represents hydrogen or a N-protecting group comprising C₁₋₄ alkyl, such as methyl, ethyl, propyl and butyl; and X is the corresponding counter-anion of said salt of choline;
and, optionally, removing off said N-protecting group of the intermediate of formula III and/or introducing said group R in said intermediate of formula III.

29. A method of preparing a substrate capable of detecting a microbial Phosphatidylcholine-specific Phospholipase C enzyme wherein said method comprises producing said substrate by incorporating therein a 3-Indoxyl choline phosphate compound as claimed in any of claims 1-5.

## Patentansprüche

1. Chromogene 3-Indoxylcholinphosphat-Verbindung der Formel (I): 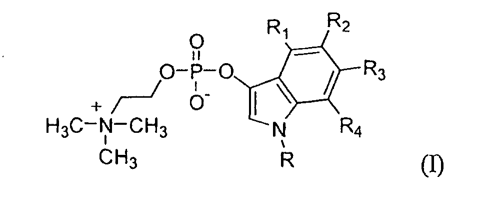 in der R gewählt ist aus Wasserstoff- und C₁ - C₄-Alkylgruppen, wie Methyl-, Ethyl-, Propyl- und Butylgruppen, und wobei R¹, R², R³ und R⁴ unabhängig voneinander gewählt sind aus Wasserstoff-, Halogen-, Cyano-, Nitro-, Carboxy-, Amino-, mit einer oder zwei C₁ - C₄-Alkylgruppen substituierten Amino-, Aminomethyl-, Hydroxy-, C₁ - C₄-Alkoxy-, Carboxyalkyl- oder Sulfonylgruppen.

2. Verbindung nach Anspruch 1, in der R Wasserstoff oder Methyl ist, R¹ Wasserstoff oder Halogen, R² Wasserstoff, Halogen, Cyano oder Nitro ist und R³ und R⁴ unabhängig voneinander Wasserstoff oder Halogenen sind.

3. Verbindung nach den Ansprüchen 1 oder 2, in der R Wasserstoff, R¹ Wasserstoff oder Chlor, R² Wasserstoff oder Brom, R³ Wasserstoff, Chlor oder Fluor und R⁴ Wasserstoffist; und worin vorzugsweise R, R¹ und R⁴ Wasserstoff bedeuten, R² Wasserstoff oder Brom und R³ Chlor ist.

4. 5-Brom-4-chlor-3-indoxyl-cholin-phosphat der Formel (IV):

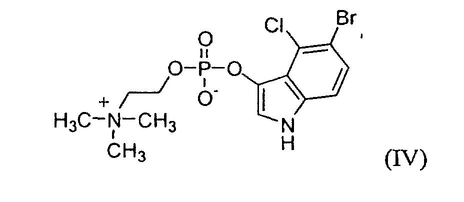

5. 3-Indoxyl-cholin-phosphat der Formel (V):

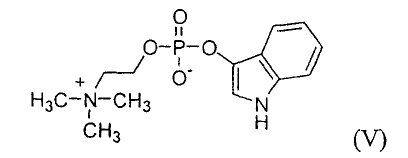

6. Substrat zum Nachweisen eines phosphatidylcholin-spezifischen Phospholipase-C-Enzyms als Anzeichen mikrobieller Aktivität; wobei das Substrat mindestens eine chromogene 3-Indoxyl-cholin-phosphat-Verbindung gemäss den Ansprüchen 1 - 5 enthält.

7. Substrat nach Anspruch 6, das zusätzlich mindestens einen Zusatz gewählt aus Serumalbumin, Tensiden und Metall-Ionen enthält.

8. Substrat nach Anspruch 6, das ausserdem mindestens ein Metall-Ion und mindestens Tensid oder Serumalbumin enthält.

9. Substrat nach den Ansprüchen 7 oder 8, bei dem das Metall-Ion ein zweiwertiges Ion von Cobalt, Mangan, Nickel, Zink, Calcium oder Magnesium ist.

10. Verfahren zum Nachweis von phosphatidylcholin-spezifischen Phospholipase-C-Enzymen mit folgende Schritten:
- Zusammenbringen einer Probe, in der das phosphatidylcholin-spezifische Phospholipase-C-Enzym vermutet wird, mit einem Substrat, das mindestens eine 3-Indoxyl-cholin-phosphat-Verbindung der Formel (I) enthält: 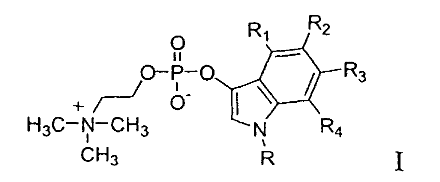 in der R gewählt ist aus Wasserstoff- und C₁ - C₄-Alkylgruppen, wie Methyl-, Ethyl-, Propyl- und Butylgruppen, und wobei R¹, R², R³ und R⁴ unabhängig voneinander gewählt sind aus Wasserstoff-, Halogen-, Cyano-, Nitro-, Carboxy-, Amino-, mit einer oder zwei C₁ - C₄-Alkylgruppen substituierten Amino-, Aminomethyl-, Hydroxy-, C₁ - C₄-Alkoxy-, Carboxyalkyl- oder Sulfonylgruppen; und wobei die Verbindung zur Spaltung durch das Enzym befähigt ist, wodurch ein Farbstoff gebildet wird; und
- Überwachung auf Farbbildung als Folge der Substanzprobe mit dem vermuteten phosphatidylcholin-spezifischen Phospholipase-C-Enzym.

11. Verfahren nach Anspruch 10, bei dem R Wasserstoff oder Methyl, R¹ Wasserstoff oder Halogenen, R² Wasserstoff, Halogen, Cyano oder Nitro ist und R³ und R⁴ unabhängig voneinander Wasserstoff oder Halogenen sind.

12. Verfahren nach den Ansprüchen 10 und 11, wobei R Wasserstoff, R¹ Wasserstoff oder Chlor, R² Wasserstoff oder Brom, R³ Wasserstoff, Chlor oder Fluor und R⁴ Wasserstoff ist; und worin vorzugsweise R, R¹ und R⁴ Wasserstoff sind, R² Wasserstoff oder Brom und R³ Chlor ist.

13. Verfahren nach Anspruch 10, wobei mindestens eine der genannten Verbindungen 5-Brom-4-chlor-3-indoxyl-cholin-phosphat ist, das der Formel (IV) entspricht:

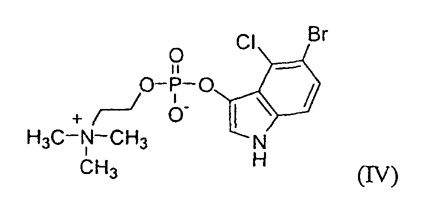

14. Verfahren nach einem der Ansprüche 10 bis 13, bei dem das Substrat ausserdem mindestens einen Zusatz gewählt aus Serumalbumin, Tensiden und Metall-Ionen enthält.

15. Verfahren nach einem der Ansprüche 10 bis 13, bei dem das Substrat ausserdem mindestens ein Metall-Ion und mindestens Tensid oder Serumalbumin enthält.

16. Verfahren nach Anspruch 14 oder 15, bei dem das Metall-Ion ein zweiwertiges Cobalt-, Mangan-, Nickel-, Zink-, Calcium- oder Magnesium-Ion ist.

17. Verfahren nach einem der Ansprüche 10 - 16, bei dem das phosphatidylcholin-spezifische Phospholipase-C-Enzym von einem Pathogen stammt, das zu der Gruppe *Clostridium perfringens, Clostridium novyi, Bacillus cereus, Bacillus thuringiensis, Pseudomonas aeruginosa, Helicobacter pylori, Legionella pneumophila, Listeria monocytogenes* und *Candida albicans* gehört.

18. Verfahren zum Nachweis mikrobieller Aktivität in einer Probe; das folgende Schritte umfasst: (i) Kombinieren der Probe mit einem Substrat, das mindestens eine Verbindung der Formel (I) nach einem der Ansprüche 1 - 5 enthält; und (ii) kolorimetrische Prüfung der mit einem Substrat kombinierten Probe.

19. Verfahren nach Anspruch 18, bei dem das Substrat ausserdem mindestens einen Zusatz gewählt aus Semmalbumin, Tensiden oder Metall-Ionen enthält, wobei das Metall-Ion vorzugsweise ein zweiwertiges Ion von Cobalt, Mangan, Nickel, Zink, Calcium oder Magnesium ist.

20. Verfahren nach den Ansprüchen 18 oder 19 zur Verwendung als Vorauswahl für den Nachweis von Bakterien der Gruppe *Clostridium perfringens, Clostridium novyi, Bacillus cereus, Bacillus thuringiensis, Pseudomonas aeruginosa, Helicobacter pylori*, *Legionella pneumophila* und *Listeria monocytogenes* sowie zum Nachweis der Hefe *Candida albicans.*

21. Ausrüstung zum Nachweis eines phosphatidylcholin-spezifischen Phospholipase-C-Enzyms als Anzeichen mikrobieller Aktivität; wobei die Ausrüstung mindestens eine Verbindung der Formel (I) nach einem der Ansprüche 1 bis 5 enthält.

22. Testverfahren zum Nachweis von PC-PLC mittels eines Substrats gemäss einem der Ansprüche 6 bis 9.

23. Testverfahren zum Nachweis der Anwesenheit pathogener Stämme von *Clostridium perfringens* in einer Probe, in der diese Stämme vermutetet werden, mittels eines Substrats gemäss einem der Ansprüche 6 bis 9.

24. Testverfahren zum Nachweis der Anwesenheit pathogener Stämme von *Clostridium perfringens* in einer Probe, in der diese Stämme vermutet werden, mittels eines Substrats, das 5-Brom-4-chlor-3-indoxyl-cholin-phosphat der Formel (IV) oder 3-Indoxyl-cholin-phosphat der Formel (V), Rinder-Serumalbumin und mindestes eine Quelle für Co²⁺-Ionen enthält.

25. Testverfahren zum Nachweis der Anwesenheit pathogener Stämme von *Bacillus cereus* oder *Bacillus thuringiensis* in einer Probe, in der solche Stämme vermutet werden, mittels eines Substrats, das 5-Brom-4-chlor-3-indoxyl-cholinphosphat der Formel (IV) oder 3-Indoxyl-cholin-phosphat der Formel (V), Rinder-Serumalbumin, Polyoxyethylensorbitanmonooleat und mindestes eine Quelle für. Mn²⁺-Ionen enthält.

26. Testverfahren zum Nachweis der Anwesenheit pathogener Stämme von *Pseudomonas aeruginosa* in einer Probe, in der solche Stämme vermutet werden, mittles eines Substrats, das 5-Brom-4-chlor-3-indoxyl-cholin-phosphat der Formel (IV) oder 3-Indoxyl-cholin-phosphat der Formel (V), Rinder-Serumalbumin, Polyoxyethylensorbitanmonooleat und mindestes eine Quelle für Zn²⁺-Ionen enthält.

27. Verfahren zur Identifizierung eines bestimmten bakteriellen Mikroorganismus, der zur Bildung eines phosphatidyl-chlolin-spezifisches Phospholipase-C-Enzyms (PC-PLC) befähigt ist, mit folgenden Schritten:
(A) Bereitstellen einer Testprobe, in der diese Mikroorganismen vermutet werden;
(B) gegebenenfalls Voranreicherung der Testprobe;
(C) Übertragen mindestens eines Teils des in Schritt (B) erhaltenen Produkts in ein Medium, das sich zum Kultivieren der Mikroorganismen eignet, wobei das Medium mindestens eine Verbindung der Formel (I) enthält, die sich verfärbt, wenn sie dem Mikroorganismus ausgesetzt wird,
(D) Kultivieren des Mediums mit dem übertragenen Teil des Produkts zur Entwicklung mindestens einer Kolonie, welche die Färbung aufweist; und
(E) Zurückbehalten mindestens eines Teils der verfärbten Kolonie zur abschliessenden Identifizierung.

28. Verfahren zur Herstellung einer chromogenen 3-Indoxyl-cholin-phosphat-Verbindung der Formel (I) 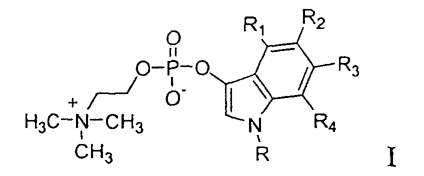 in der R gewählt ist aus Wasserstoff- und C₁ - C₄-Alkylgruppen, wie Methyl-, Ethyl-, Propyl- und Butylgruppen, und wobei R¹, R², R³ und R⁴ unabhängig voneinander gewählt sind aus Wasserstoff-, Halogen-, Cyano-, Nitro-, Carboxy-, Amino-, mit einer oder zwei C₁ - C₄-Alkylgruppen substituierten Amino-, Aminomethyl-, Hydroxy-, C₁ - C₄-Alkoxy-, Carboxyalkyl- oder Sulfonylgruppen; durch Umsetzung eines entsprechenden Indoxy-3-dichlorphosphats der Formel (II) mit einem Cholinsalz zur Bildung einer intermediären Verbindung der Formel (III) gemäss folgender Reaktion: 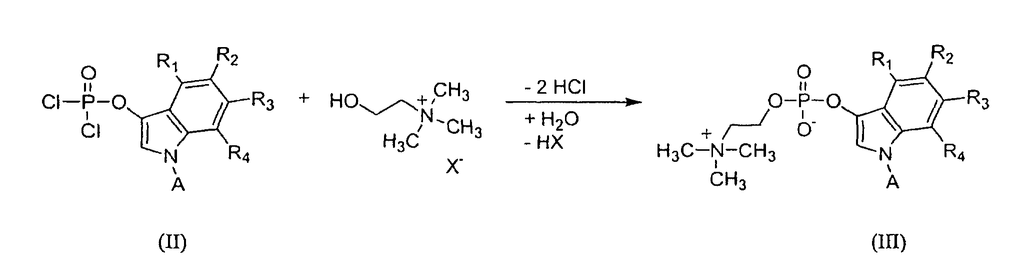 wobei A Wasserstoff oder eine N-Schutzgruppe ist, die eine C₁ - C₄-Alkylgruppe, wie Methyl, Ethyl, Propyl und Butyl ist, und X das entsprechende Gegenanion des Cholinsalzes ist; und gegebenenfalls Entfernung der N-Schutzgruppe der intermediären Verbindung der Formel (III) und/oder Einführung der Gruppe R in die intermediäre Verbindung der Formel (III).

29. Verfahren zur Herstellung eines Substrats, das zum Nachweis eines mikrobiellen phosphatidylcholin-spezifischen Phospholipase-C-Enzyms befähigt ist, wobei das Herstellungsverfahren zur Erzeugung des Substrats das Einführen einer 3-Indoxyl-cholin-phosphatverbindung gemäss den Ansprüchen 1-5 umfasst.

## Revendications

1. Composé phosphate de 3-indoxyl choline chromogène de formule I: 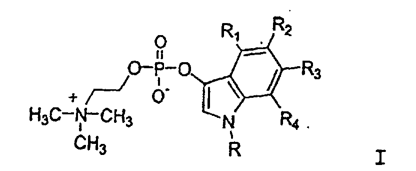 dans laquelle R est choisi dans le groupe constitué par l'hydrogène et un groupe alkyle en C₁ à C₄, comme méthyle, éthyle, propyle et butyle alors que R¹, R², R³ et R⁴ sont indépendamment choisis dans le groupe constitué par l'hydrogène, un halogène, un groupe cyano, nitro, carboxy, amino, amino substitué par un ou deux groupes alkyles en C₁ à C₄, aminométhyle, hydroxy, alcoxy en C₁ à C₄, carboxyalkyle et sulfonyle.

2. Composé selon la revendication 1, dans lequel R est choisi dans le groupe constitué par l'hydrogène et un groupe méthyle; R¹ est choisi dans le groupe constitué par l'hydrogène et un halogène; R² est choisi dans le groupe constitué par l'hydrogène, un halogène, un groupe cyano, nitro; et R³ et R⁴ sont indépendamment choisis dans le groupe constitué par l'hydrogène et un halogène.

3. Composé selon la revendication 1 ou 2, dans lequel R est de l'hydrogène, R' est choisi parmi l'hydrogène et le chlore, R² est choisi parmi l'hydrogène et le brome, R³ est choisi parmi l'hydrogène, le chlore et le fluor, et R⁴ est de l'hydrogène; et de préférence dans lequel R, R¹ et R⁴ sont de l'hydrogène, R² est de l'hydrogène ou du brome, et R³ est du chlore.

4. Phosphate de 5-bromo-4-chloro-3-indoxyl choline représenté par la formule (IV):

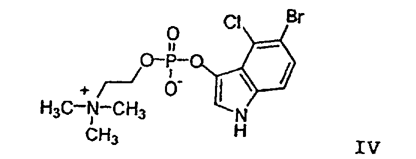

5. Phosphate de 3-indoxyl choline représenté par la formule (V):

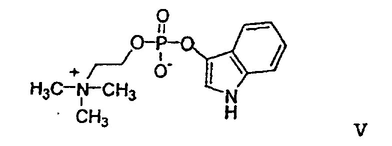

6. Substrat pour détecter une enzyme phospholipase C spécifique à la phosphatidylcholine en tant qu'indication d'une activité microbienne; ledit substrat comprenant au moins un composé phosphate de 3-indoxyl choline chromogène tel que défini dans l'une quelconque des revendications 1 à 5.

7. Substrat selon la revendication 6, comprenant de plus au moins un additif choisi dans le groupe constitué par la sérum albumine, les agents tensioactifs et les ions métalliques.

8. Substrat selon la revendication 6, comprenant de plus au moins un ion métallique et au moins un élément choisi dans le groupe constitué par les agents tensioactifs et la sérum albumine.

9. Substrat selon la revendication 7 ou 8, dans lequel ledit ion métallique est un ion divalent de cobalt, manganèse, nickel, zinc, calcium ou magnésium.

10. Procédé de détection d'une enzyme phospholipase C spécifique à la phosphatidylcholine, comprenant les étapes consistant:
à mettre en contact un échantillon suspecté de contenir ladite enzyme phospholipase C spécifique à la phosphatidylcholine avec un substrat contenant au moins un composé phosphate de 3-indoxyl choline de formule I: 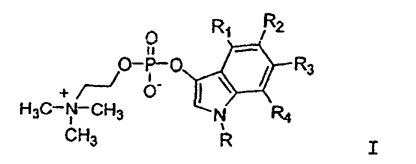 dans laquelle R est choisi dans le groupe constitué par l'hydrogène et un groupe alkyle en C₁ à C₄, tel que méthyle, éthyle, propyle et butyle, alors que R¹, R², R³ et R⁴ sont indépendamment choisis dans le groupe constitué par l'hydrogène, un halogène, un groupe cyano, nitro, carboxy, amino, amino substitué par un ou deux groupes alkyles en C₁ à C₄, aminométhyle, hydroxy, alcoxy en C₁ à C₄, carboxyalkyle et sulfonyle; et ledit composé étant susceptible d'être clivé par ladite enzyme en donnant un colorant; et
à suivre la formation de couleur sous l'effet dudit échantillon suspecté de contenir ladite enzyme phospholipase C spécifique à la phosphatidylcholine.

11. Procédé selon la revendication 10, dans lequel R est choisi dans le groupe constitué par l'hydrogène et un groupe méthyle; R¹ est choisi dans le groupe constitué par l'hydrogène et un halogène; R² est choisi dans le groupe constitué par l'hydrogène, un halogène, un groupe cyano, nitro; et R³ et R⁴ sont indépendamment choisis dans le groupe constitué par l'hydrogène et un halogène.

12. Procédé selon la revendication 10 ou 11, dans lequel R est de l'hydrogène, R¹ est choisi parmi l'hydrogène et le chlore, R² est choisi parmi l'hydrogène et le brome, R³ est choisi parmi l'hydrogène, le chlore et le fluor, et R⁴ est de l'hydrogène; et de préférence dans lequel R, R¹ et R⁴ sont de l'hydrogène, R² est de l'hydrogène ou du brome et R³ est du chlore.

13. Procédé selon la revendication 10, dans lequel ledit au moins un composé est le phosphate de 5-brome-4-chloro-3-indoxyl choline représenté par la formule IV:

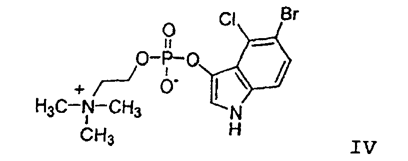

14. Procédé selon l'une quelconque des revendications 10 à 13, dans lequel ledit substrat comprend en outre au moins un additif choisi dans le groupe constitué par la sérum albumine, les agents tensioactifs et les ions métalliques.

15. Procédé selon l'une quelconque des revendications 10 à 13, dans lequel ledit substrat comprend en outre au moins un ion métallique et au moins un élément choisi dans le groupe constitué par les agents tensioactifs et la sérum albumine.

16. Procédé selon la revendication 14 ou 15, dans lequel ledit ion métallique est un ion divalent de cobalt, manganèse, nickel, zinc, calcium ou magnésium.

17. Procédé selon l'une quelconque des revendications 10 à 16, dans lequel ladite enzyme phospholipase C spécifique à la phosphatidylcholine provient d'un agent pathogène choisi dans le groupe constitué par *Clostridium perfringens, Clostridium novyi, Bacillus cereus, Bacillus thuringiensis, Pseudomonas aeruginosa, Helicobacter pylori, Legionella pneumophila, Listeria monocytogenes* et *Candida albicans.*

18. Procédé de détection d'une activité microbienne dans un échantillon; ledit procédé comprenant les étapes consistant (i) à combiner ledit échantillon avec un substrat comprenant au moins un composé de formule I tel que défini dans l'une quelconque des revendications 1 à 5; et (ii) à inspecter ledit échantillon combiné avec ledit substrat par un moyen colorimétrique.

19. Procédé selon la revendication 18, dans lequel ledit substrat comprend en outre au moins un additif choisi dans le groupe constitué par la sérum albumine, les agents tensioactifs et les ions métalliques, de préférence ledit ion métallique est un ion divalent de cobalt, manganèse, nickel, zinc, calcium ou magnésium.

20. Procédé selon la revendication 18 ou 19, pour l'utilisation en tant que test de dépistage dans la détection de bactéries du groupe incluant *Clostridium perfringens, Clostridium novyi, Bacillus cereus, Bacillus thuringiensis*, *Pseudomonas aeruginosa, Helicobacter pylori, Legionella pneumophila* et *Listeria monocytogenes* ainsi que dans la détection de la levure *Candida albicans.*

21. Nécessaire pour détecter une enzyme phospholipase C spécifique à la phosphatidylcholine en tant qu'indication d'une activité microbienne; ledit nécessaire incluent au moins un composé de formule I tel que défini dans l'une quelconque des revendications 1 à 5.

22. Procédé d'essai pour détecter une enzyme PC-PLC au moyen d'un substrat tel que défini dans l'une quelconque des revendications 6 à 9.

23. Procédé d'essai pour détecter la présence de souches pathogènes de *Clostridium perfringens* dans un échantillon suspecté de contenir de telles souches au moyen d'un substrat tel que défini dans l'une quelconque des revendications 6 à 9.

24. Procédé d'essai pour détecter la présence de souches pathogènes de *Clostridium perfringens* dans un échantillon suspecté de contenir de telles souches au moyen d'un substrat comprenant du phosphate de 5-bromo-4-chloro-3-indoxyl choline représenté par la formule (IV) ou du phosphate de 3-indoxyl choline représenté par la formule (V), de la sérum albumine bovine et au moins une source d'ions Co²⁺.

25. Procédé d'essai pour la détection de la présence de souches pathogènes de *Bacillus cereus* ou de *Bacillus thuringiensis* dans un échantillon suspecté de contenir de telles souches au moyen d'un substrat comprenant du phosphate de 5-bromo-4-chloro-3-indoxyl choline représenté par la formule (IV) ou du phosphate de 3-indoxyl choline représenté par la formule (V), de la sérum albumine bovine, du monooléate de polyoxyéthylène sorbitane et au moins une source d'ions Mn²⁺.

26. Procédé d'essai pour la détection de la présence de souches pathogènes de *Pseudomonas aeruginosa* dans un échantillon suspecté de contenir de telles souches au moyen d'un substrat comprenant du phosphate de 5-bromo-4-chloro-3-indoxyle représenté par la formule (IV) ou du phosphate de 3-indoxyl choline représenté par la formule (V), de la sérum albumine bovine, du monooléate de polyoxyéthylène sorbitane et au moins une source d'ions Zn²⁺.

27. Procédé d'identification d'un micro-organisme bactérien d'intérêt, qui est apte à produire une enzyme phospholipase C spécifique à la phosphatidylcholine (PC-PLC), comprenant les étapes consistant:
(A) à fournir un échantillon d'essai suspecté de contenir ledit micro-organisme d'intérêt;
(B) de préférence à soumettre ledit échantillon d'essai à une étape de bouillon d'enrichissement;
(C) à transférer une partie, au moins, dudit échantillon d'essai ou du produit obtenu dans l'étape (B) à un milieu approprié pour cultiver ledit micro-organisme; ledit milieu contenant au moins un composé de formule I apte à produire une couleur lorsqu'il est exposé audit micro-organisme;
(D) à cultiver ledit milieu avec ladite partie transférée pour développer au moins une colonie présentant ladite couleur; et
(E) à récupérer une partie, au moins, de ladite colonie colorée pour l'identification finale.

28. Procédé de préparation d'un composé phosphate de 3-indoxyl choline chromogène de formule I 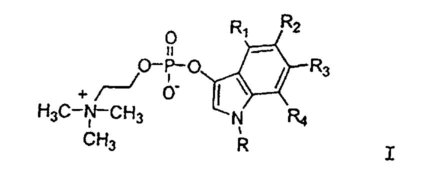 dans laquelle R est choisi dans le groupe constitué par l'hydrogène et un groupe alkyle en C₁ à C₄, tel que méthyle, éthyle, propyle et butyle, alors que R¹, R², R³ et R⁴ sont indépendamment choisis dans le groupe constitué par l'hydrogène, un halogène, un groupc cyano, nitro, carboxy, amino, amino substitué par un ou deux groupes alkyles en C₁ à C₄, aminométhyle, hydroxy, alcoxy en C₁ à C₄, carboxyalkyle et sulfonyle;
comprenant les étapes consistant à faire réagir un 3-dichlorophosphate d'indoxyle correspondant de formule II avec un sel de choline afin d'obtenir un composé intermédiaire III d'après la réaction: 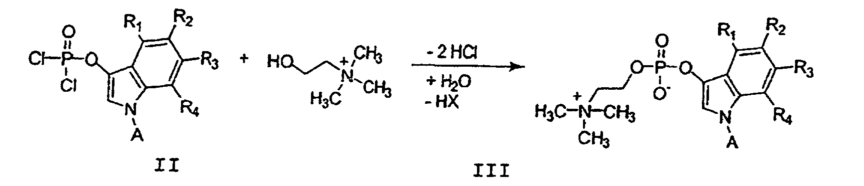 dans laquelle A représente de l'hydrogène ou un groupe protégeant N comprenant un groupe alkyle en C₁ à C₄, tel que méthyle, éthyle, propyle et butyle; et X est le contre-anion correspondant dudit sel de choline;
et, éventuellement, à éliminer ledit groupe protégeant N du produit intermédiaire de formule III et/ou introduire ledit groupe R dans ledit produit intermédiaire de formule III.

29. Procédé de préparation d'un substrat apte à détecter une enzyme phospholipase C spécifique à la phosphatidylcholine microbienne, ledit procédé comprenant la production dudit substrat en incorporant dans celui-ci un composé phosphate de 3-indoxyl choline tel que revendiqué dans l'une quelconque des revendications 1 à 5.
